# EUROPEAN PATENT SPECIFICATION

(11) **EP 1 594 752 B1**
(45) Date of publication and mention of the grant of the patent: **28.03.2007**
(21) Application number: 03814045.5
(22) Date of filing: 16.12.2003
(51) Int. Cl.: B65D 33/14, F42B 3/087

(54) **ATTACHABLE BAGS**
BEFESTIGBARE BEUTEL
SACS POUVANT ETRE ATTACH ES

(30) Priority: 16.12.2002 US 434085 P; 12.08.2003 US 494653 P; 12.08.2003 US 494659 P
(43) Date of publication of application: 16.11.2005
(73) Proprietor: VELCRO INDUSTRIES B.V., Curacao (AN)
(72) Inventor: KINGSFORD, Howard, A., Amherst, NH 03031 (US); ISABELLE, Holly, Deerfield, NH 03037 (US); PROVOST, George, A., Litchfield, NH 03052 (US); SHEPARD, William, H., Amherst, NH 03031 (US)
(74) Representative: Müller-Boré & Partner Patentanwälte
(86) International application number: PCT/US2003/040035
(87) International publication number: WO 2004/058584

(56) References cited:
- DE-U- 29 920 079
- US-A- 3 203 551
- US-A- 3 999 483
- US-A- 5 020 711
- US-B1- 6 202 260

## Description

### TECHNICAL FIELD

This invention relates to bags defining interior compartments and securable to other objects.

### BACKGROUND

Objects are often packaged in bags, and it is occasionally necessary to secure such packaged objects to other objects.

### SUMMARY OF THE INVENTION

According to one aspect of the invention, a bag includes a bag body defining therein a compartment accessible through an opening at one end of the bag body, and, secured to the bag body along one edge thereof and extending therefrom to a free distal end, a flexible strap of width sufficient to span at least a majority of the compartment. The strap carries an array of fastener elements on an opposite side, each fastener element having a stem extending integrally from a band of resin film extending across the strap. The strap is of sufficient length to wrap about an object, with the strap overlapping the bag body to engage exposed fibers of the bag with the fastener elements, to secure the bag to the object.

In some preferred configurations, the band of resin encapsulates surface features of the strap to form an inseparable laminate. Such encapsulation may be obtained by integrally forming the band of resin from molten resin applied directly to the strap, for example, such that the resin, as it solidifies, encapsulates surface features, such as asperities or fibers, of the strap.

In some other arrangements, the band of resin is an integral region of the strap, the strap being a unitary sheet of the resin and extending beyond the array of fastener elements. One disclosed method of forming this structure involves calendering heated resin to form both the band of resin and the remainder of the strap.

In some cases, the strap extends beyond the edge of the bag a distance greater than about twice a width of the bag measured from the same edge in opposite direction.

In some embodiments, the strap consists essentially of a sheet of loop material with the band of resin extending thereacross.

In some examples, the bag body is secured to the strap along multiple edges of the bag body.

In some cases the strap is an integral extension of one side of the bag body.

For some applications, the bag body includes a releasable closure extending along the opening. The closure may be a rib-and-groove closure, or a touch fastener closure, for example.

In some configurations, the opening faces the strap. In some others, the opening extends along one side edge of the strap.

Preferably, the strap is resiliently, stretchable in a longitudinal sense.

In some embodiments, the fastener elements are disposed in a discrete band adjacent the free end of the strap. Preferably, the discrete band extends across the width of the strap. In some cases, the strap includes a graspable, non-fastening region at its free end, beyond the band of fastener elements.

Preferably, the compartment is defined fully within the width of the strap, such that the strap envelops the compartment when overlapped across the bag body. This can be very useful for protecting objects contained within the compartment when wrapped, or for ensuring finn engagement between the bag body and a wrapped object, or for maintaining pressure between the bag body and wrapped object.

The wrap-bag can be useful for many medical applications in which the bag is wrapped around a patient, such as for thermal therapy, as described in a U.S. provisional application by us being filed concurrently herewith and entitled "Medical Wraps," the contents of which are hereby incorporated by reference.

The wrap-bag can also be useful for non-medical applications, such as by placing ice or other cooling substance in the compartment and wrapping the bag about a drinking container. In other cases, the wrap-bag is wrapped about a cement column for holding, forming and accelerating setting of repair materials or wrapped about a post or structural joint for positioning a shaped explosive charge for demolition. Various similar uses will be apparent.

According to other aspects of the invention, various methods of forming wrap-bags, and for forming composite materials from which wrap-bags can be made, are provided.

Another aspect of the invention features a method of releasably securing one or more relatively small components to a relatively larger object. The method includes placing the components in the compartment of the above-described wrap-bag, and wrapping the wrap-bag about the object in overlapping manner, releasably securing the fastener elements of the wrap-bag to the fibers of the wrap-bag, to hold the wrap-bag to the object.

In one example, the relatively large object is a chassis, and the components are to be later assembled to the chassis.

In another example, the relatively large object is to be detonated, and the components comprise one or more explosive charges.

Another aspect of the invention features methods of making bags with integral straps, such as by the continuous forming methods disclosed herein.

The wrap-bag described herein can provide several advantages in use. For example, the substantial width of the strap portion of the bag, as compared with the bag compartment, can enable the strap to be readily wrapped about the bag body in a way that substantially envelops a major portion of the contents contained within the bag body. In this manner, the wrap-bag can be easily wrapped so as to create a sustained pressure between the bag body and wrapped object, as well as intimate engagement between them. This can be effective in several medical uses, as well as in applications such as demolition or repair, when it is critical to hold an explosive charge or repair materials closely to their intended subject.

Various constructions of the wrap-bag enable it to be wrapped about an object in such a way that only two hands are required, and without having to hold the bag in place while adhesive tape is cut and arranged. In some in-field applications, such as emergency medical treatment, this is seen to be particularly advantageous.

The methods and materials described below enable the wrap-bag to be formed at extremely low cost, enabling its use as a disposable product for several applications.

Details of embodiments are set forth in the accompanying drawings and the description below. Other features, objects, and advantages will be apparent from the description and drawings.

### DESCRIPTION OF DRAWINGS

FIG 1 is a cross section view of a sealable bag with an integral strap for wrapping the bag about an object.
FIG. 2 is a plan view of the bag of FIG. 1.
FIG. 3 is a diagrammatic, highly magnified cross-sectional view of the wrapping of FIG. 1 showing the flexible wrapping wrapped about an object, positioning the filled pouch with respect to it.
FIG. 4 is a diagrammatic plan view, FIG. 5 is a cross section view, and FIG. 6 a diagrammatic end view, of a calender-forming-and-uniting machine producing a continuous composite, flexible web material from which wrap-bags are formed.
FIG. 7 is a perspective view of a flat bag making machine for making the wrap-bags of FIG. 1.
FIG. 8 is a cross section view of a self securing wrap-bag having a closure.
FIG. 9 is a plan view of the wrap-bag of FIG. 8.
FIG. 10 is a perspective view of a flat bag making machine for making the wrap-bags of FIG. 8.
FIG. 11 is a side view of a wrap-bag.
FIG. 12 is a highly magnified diagrammatic view of one embodiment of a hook fastener portion of a wrap-bag.
FIG. 13 is a further magnified view of one useful form of hook.
FIGs. 14 and 15 are views of a calender-forming and molding machine producing a continuous composite, flexible web material; and FIGs. 16 and 17 are views of a machine performing the functions of FIG. 14 while producing a single continuous, composite sheet having two preformed fastener components, shown by FIG. 18 and a molded fastener component, united in situ as a composite.
FIG. 19 is a diagrammatic cross-sectional view (thicknesses grossly exaggerated) of another machine forming a material similar to that produced by the machine of FIGs. 16 and 17, in this case the hook and loop bands residing on opposite sides of the resultant composite.
FIGs. 20 and 21 are diagrammatic plan and end views of a machine forming a continuous, composite flexible material having similarities to that formed in FIG. 16, but with much wider bands of hooks and loops. FIG. 20 is a magnified partial cross-section taken parallel to the axis of the forming roll showing a formation on the mold roll for defining a fold axis.
FIG. 23 is a cross section view taken on line 23-23 of FIG. 20.

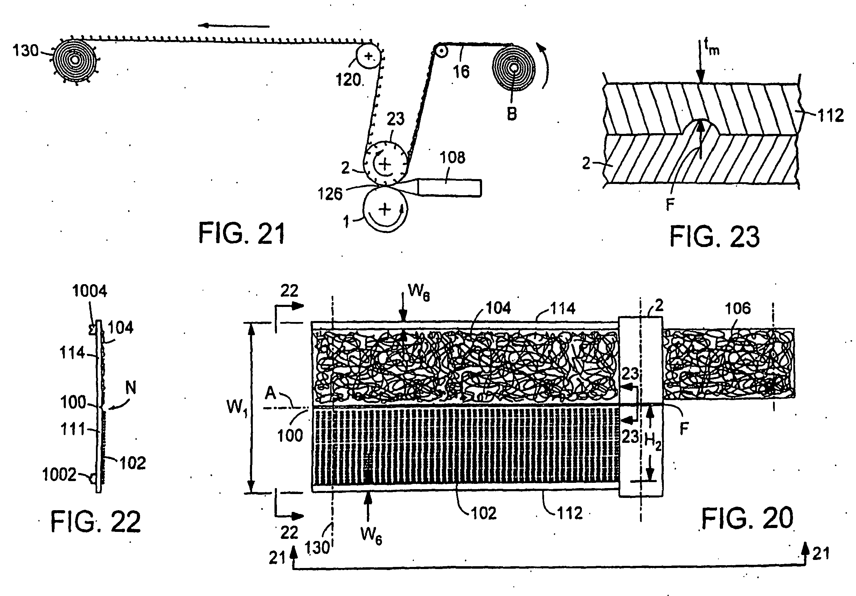

FIG. 22 is a transverse cross-section taken on line 22-22 of FIG. 20.

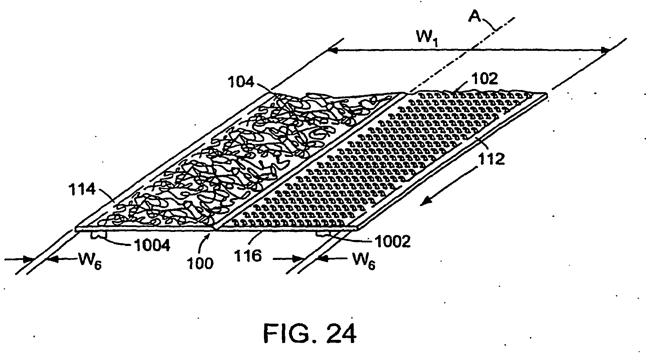

FIG. 24 is a perspective view of the sheet-form material resulting from the continuous calender roll process illustrated in FIGs. 20 and 21.

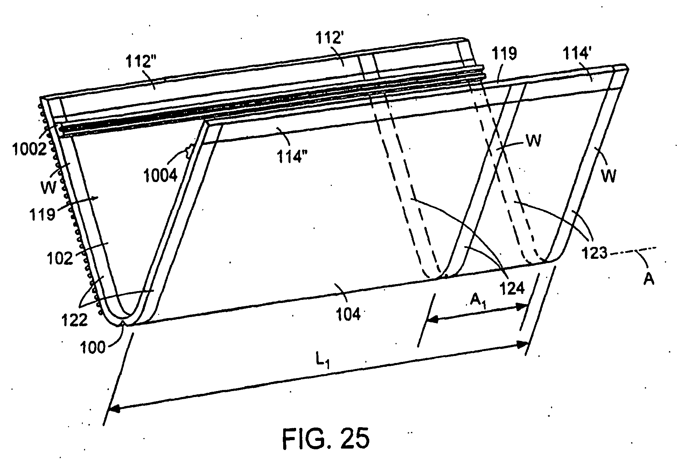

FIG. 25 is a diagrammatic perspective view with thicknesses exaggerated of the material of FIG 24 while it is being partially folded.

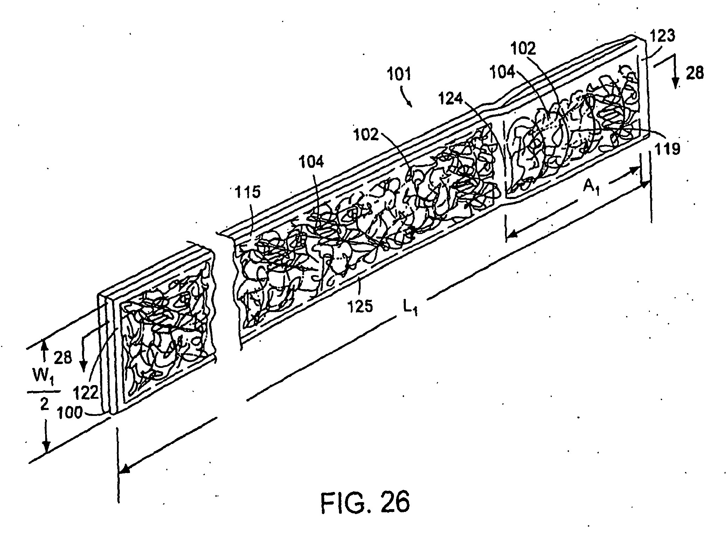

FIG. 26 is a perspective view of a flexible wrap-bag with a formed pouch made of the material of FIG. 25.

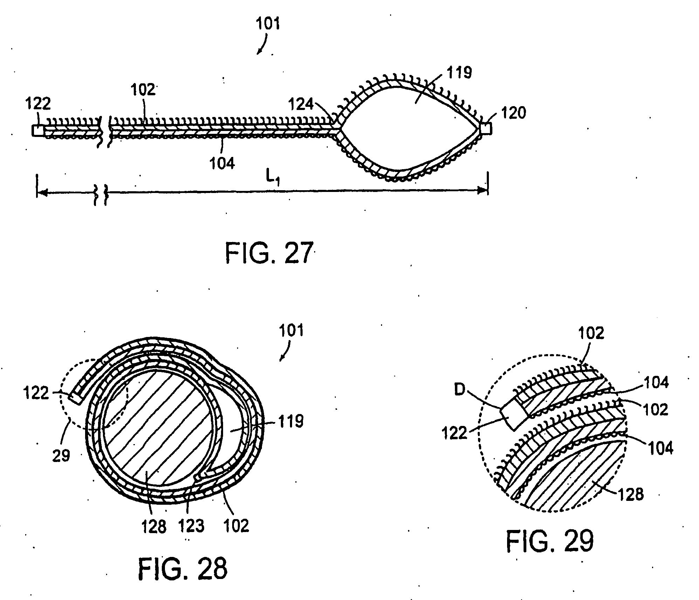

FIG. 27 is an edge view of the embodiment of FIG. 26, showing a bulge attributable to a filled pouch.
FIG. 28 is a diagrammatic, highly magnified cross-sectional view of the embodiment of FIG. 26 taken on line 28-28 showing the flexible wrap-bag wrapped about an object, positioning the filled pouch with respect to it.
FIG. 29 is a further magnified diagrammatic view of the end fastening portion of FIG. 28.

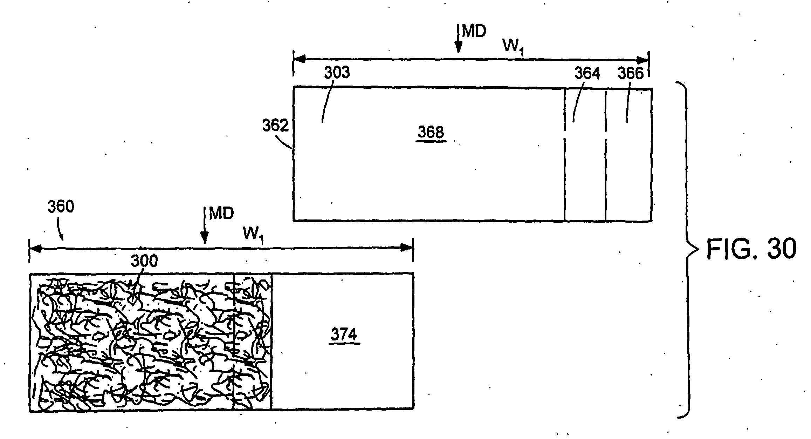

FIG. 30 is a plan view of two composite sheets of preformed starting material with engageable hooks and loops respectively.

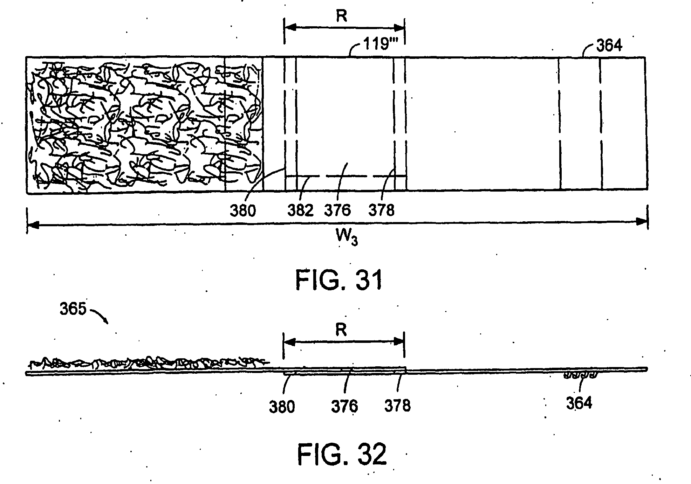

FIG. 31 is a plan view of the two sheets of FIG. 30 joined together.
FIG. 32 is an edge view of the joined sheets of FIG. 31.

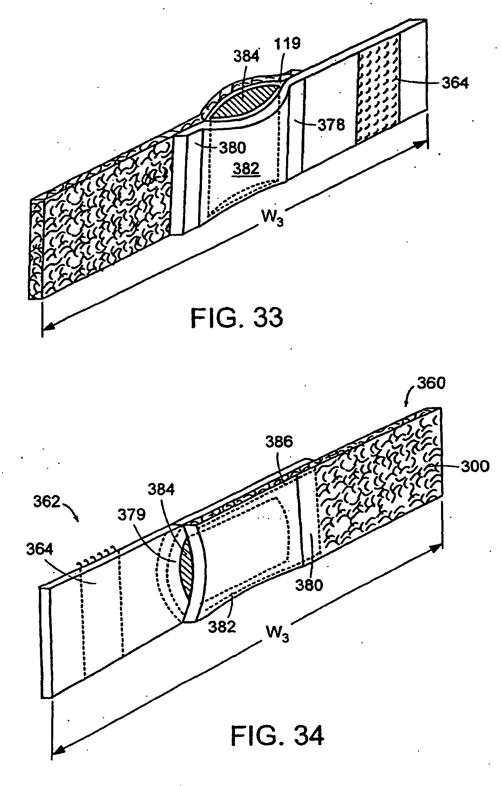

FIG. 33 is a perspective view of one embodiment having the general construction of FIG. 32.
FIG. 34 is a perspective view of another embodiment having the general construction of FIG. 32.

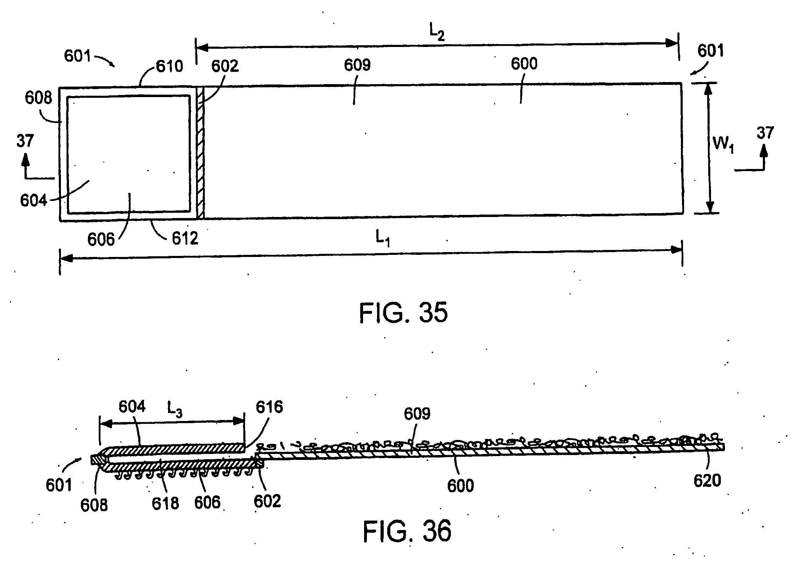

FIG. 35 is a plan view of a wrap-bag of another construction with an empty pouch.
FIG. 36 is a cross section view of the wrap-bag of FIG. 35.

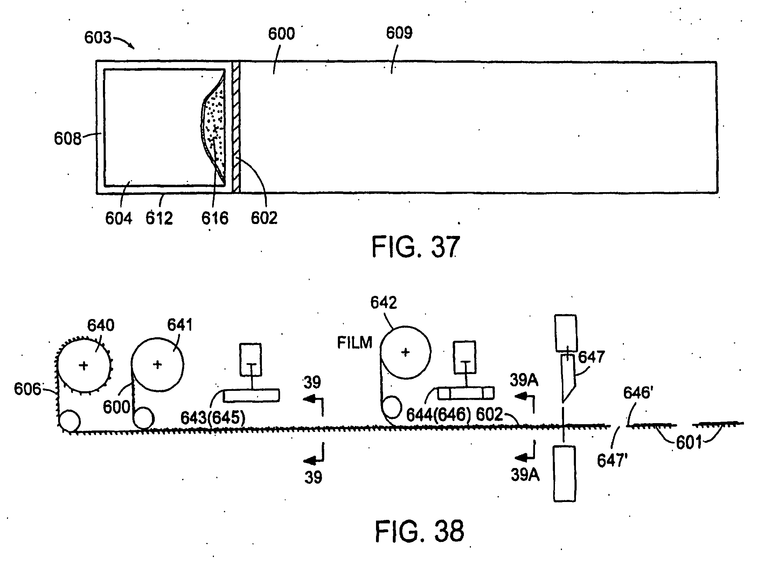

FIG. 37 is a plan view of the wrap-bag of FIG. 35 with a filled pouch.
FIG. 38 is a side view of a system for making the wrap-bag of FIG. 35.

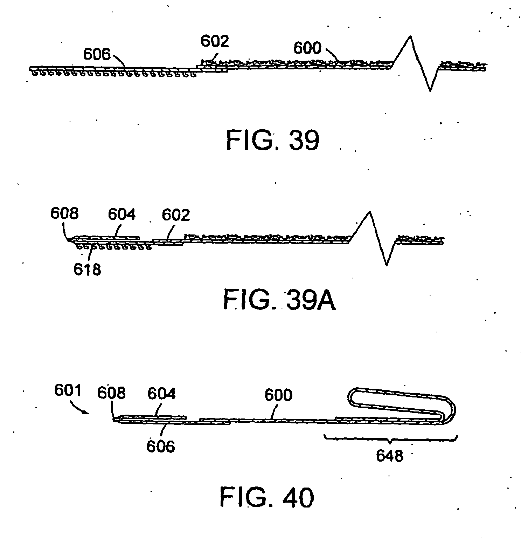

FIG. 39 is a cross section view of the continuous web in an intermediate step in the process performed by the system of FIG. 38.
FIG 39A is a cross section view of the continuous web in the last step of the process performed by the system of FIG. 38 before cutting off individual wrap-bags.
FIG. 40 is a cross section view of another form of the continuous web in the last step of the process performed by the system of FIG. 38 before cutting off individual wrap-bags.

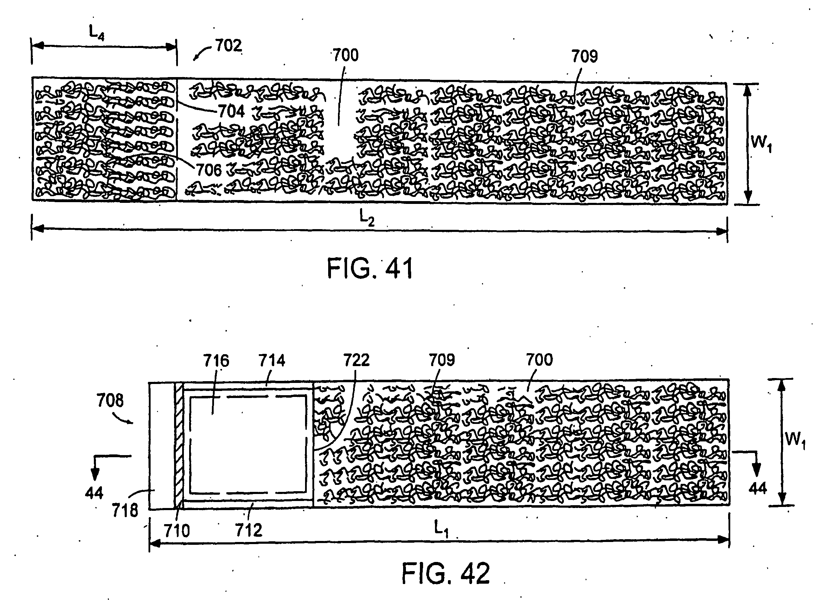

FIG. 41 is a plan view of non-woven loop material to be folded to form a wrap-bag.
FIG. 42 is a plan view of a wrap-bag constructed from the material of FIG 41.

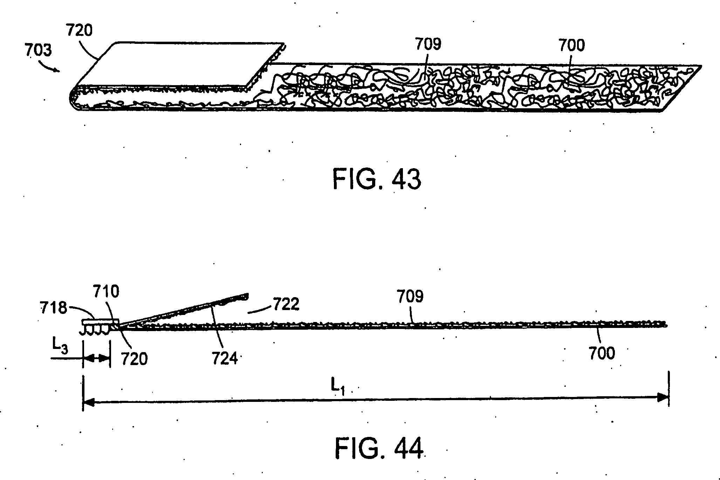

FIG 43 is a view of the material of FIG. 41 folded to form a wrap-bag with a pouch.
FIG. 44 is a cross section view of the wrap-bag of FIG. 42.

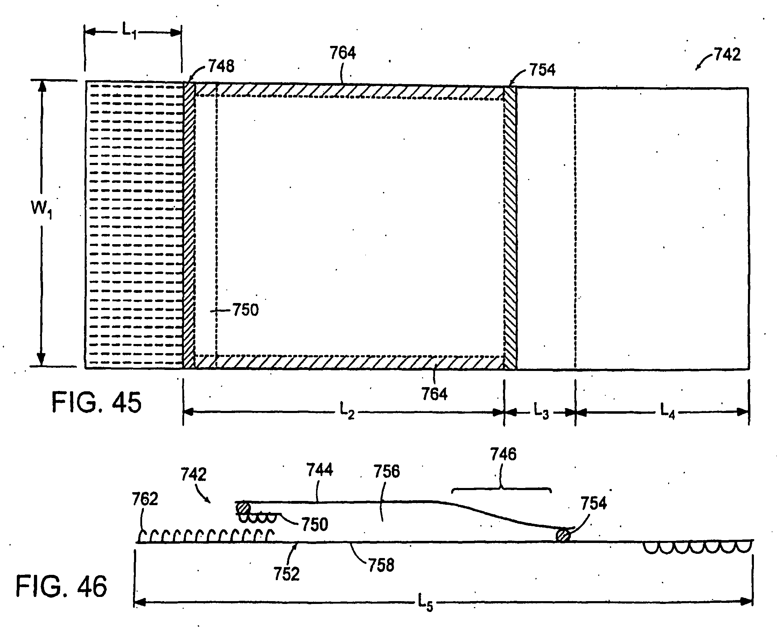

FIG. 45 is a plan view of a wrap-bag of another construction.
FIG. 46 is a cross section view of the wrap-bag shown in FIG. 45.

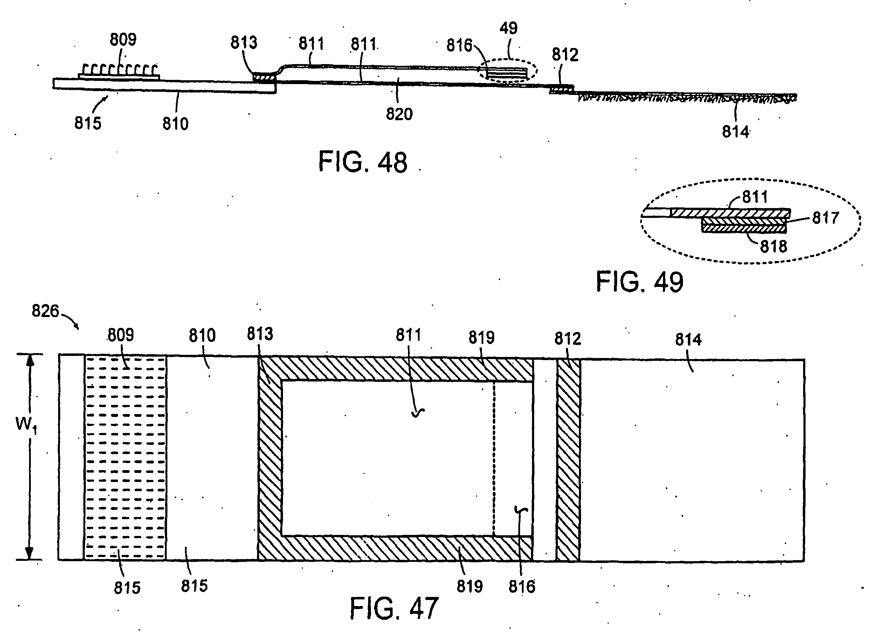

FIG. 47 is a plan view of a wrap-bag of another construction.
FIG. 48 is a cross section view of the wrap-bag shown in FIG. 47.
FIG. 49 is a magnification of a sealing assembly at the edge of the pouch flap of the wrap-bag shown in FIG. 47.

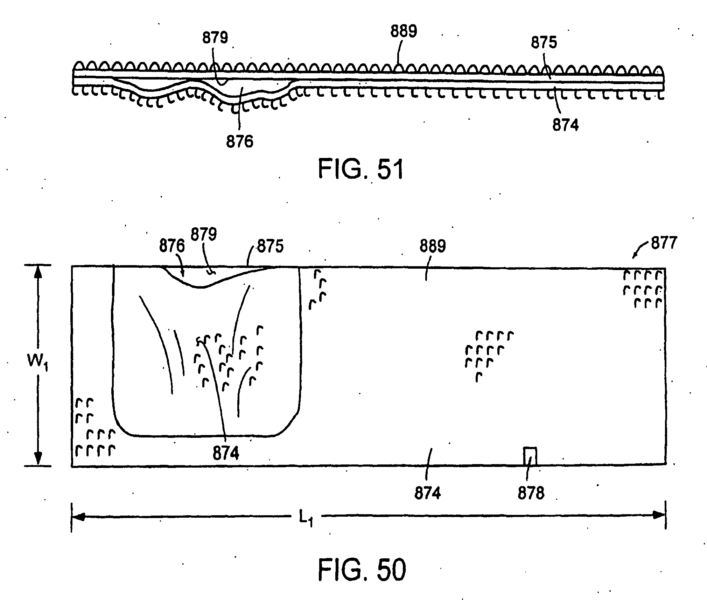

FIG. 50 is a plan view of a wrap-bag of another construction.
FIG. 51 is a side view of the wrap-bag shown in FIG. 50.

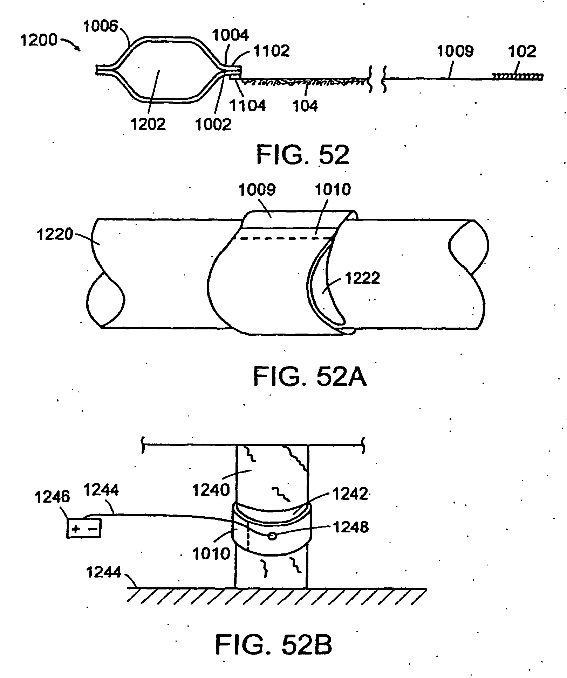

FIG. 52 is a cross section view of a wrap-bag.
FIG. 52A is a view of a wrap-bag secured to a chassis and carrying parts associated with the chassis.
FIG**.** 52B is a view of a wrap-bag secured to a cement column and carrying a shaped explosive charge.

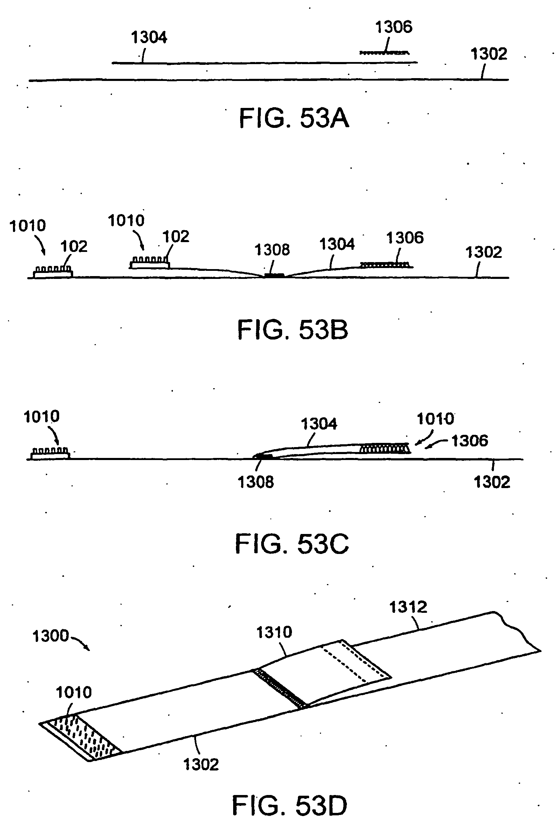

FIGs. 53A-53C are cross-sectional views of the wrap-bag shown in perspective view in FIG. 53D, at different stages of the manufacture of the wrap-bag.

Like reference symbols in the various drawings indicate like elements.

### DESCRIPTION OF EMBODIMENTS

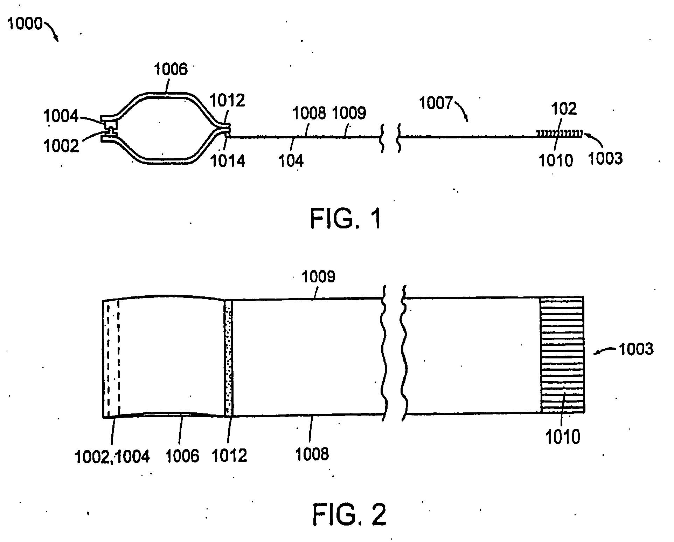

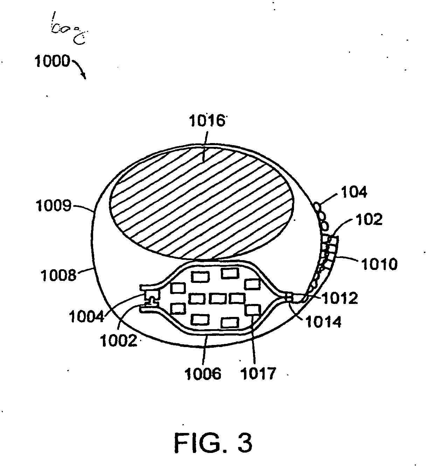

Referring to FIGs. 1 and 2, a wrap-bag 1000 uses a rib 1002 fastener and a groove 1004 fastener to seal ice in a bag 1006 permanently joined to a composite material 1007 in the form of a strap 1009 that extends to a free distal end 1003. The composite material 1007 includes a non-woven hook-engageable loop material 1008 having loops 104 on one side, and a discrete band 1010 of hooks 102 on the other side, for securing the bag when wrapped around another object 1016 in overlapping manner (FIG. 3). In some cases, loops 104 are present across the entire surface of material 1008. An example of material 1008 is an elastomeric non-porous non-woven loop material available from Tredegar Film Products of Richmond, VA. Material 1008 can also be inelastic.

Weld 1012 joins bag 1006 to non-woven loop material 1008. Non-woven loop material 1008 with hook band 1010 is made with weld bead 1014. Hook band 1010 is formed in the machine direction from an extruded band of resin laminated *in situ* directly to the surface of material 1008, and molded to form hooks or hook preforms by mold cavities in a molding roll, as disclosed in Kennedy et al., U.S. 5,260,015. Weld bead 1014 is formed at the same time in the machine direction from an extruded band of resin laminated *in situ* directly to the surface of material 1008, and molded into weld bead 1014 by a mold cavity in the molding roll. Wrap-bag 1000 is then made by welding a plastic bag with rib and groove fasteners to composite material 1007 at weld bead 1014. Wrap-bag 1000 can also be made with a folded plastic sheet having continuous rib and groove fastener strips in a continuous process using flat bag making machinery.

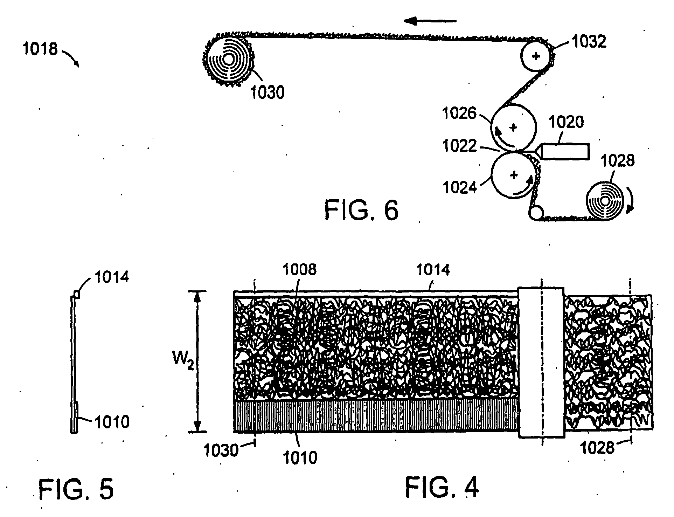

An apparatus 1018 and continuous process for making composite material 1007 is described with reference to FIGs. 4, 5, and 6. Referring particularly to FIG. 6, extruder 1020 provides to the nip 1022 a molten strip of resin having a width corresponding to a width of a desired band 1010 of molded hooks 102. Completion of the *in situ* lamination is achieved by the pressure of the calender nip 1022 formed by pressure roll 1024 and mold roll 1026. The molten strip of resin applied by extruder 1020 enters mold cavities in mold roll 1026, forming hook band 1010 that includes hooks 102 or hook preforms molded integrally with a base resin layer. A web of loop material 1008 is supplied by roll 1028. The base resin layer is *in situ* laminated to the loop web by the action of the calendar nip 1022, thereby encapsulating surface features of the loop web to securely and permanently join the materials. At the same time, resin of another band of resin applied by extruder 1020 enters mold cavities in mold roll 1026, forming weld bead 1014 that is *in situ* laminated to the loop web 1008 by the action of the calender nip 1022. After cooling, the finished wrapping material is removed from mold roll 1026 and rolled up in supply roll 1030, in which form the finished composite material 1007 is delivered to a wrapping forming machine.

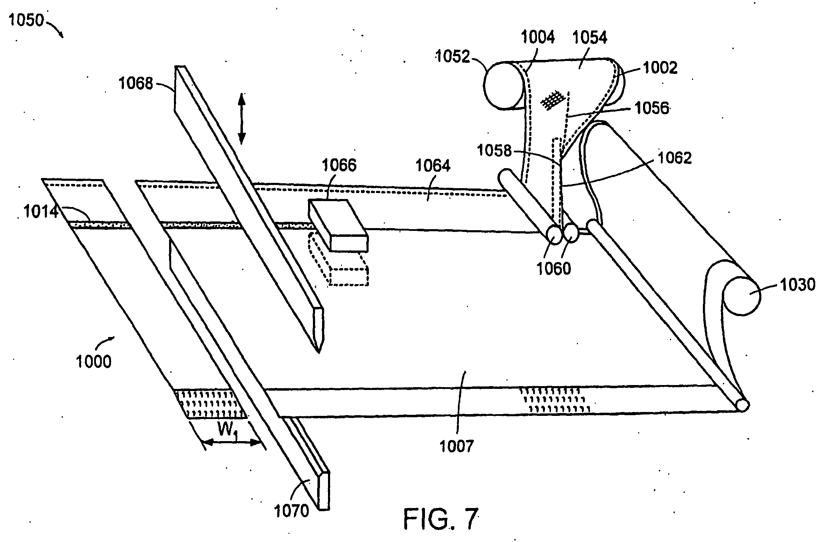

FIG. 7 illustrates a machine 1050 and method for making the wrap-bag 1000 described above. Plastic film 1054 is supplied as a roll 1052 with rib 1002 and groove 1004 fasteners welded previously to or integrally formed with the film 1054 using techniques such as extrusion. Roll 1052 of continuous plastic film 1054 is positioned such that a fold in plastic film 1054 is managed at a center fold line 1056. A folding bar or board 1058 is provided. Rollers 1060 pinch the fold 1062 just after the fold plate or folding board 1058 creates the center fold 1056. Folded sheet 1064 enters into the flat bag sealing machine 1050 on top of non-woven loop material 1008 with hook strip 1010 and weld bead 1014 to form the wrapping 1000. Typical flat bag-sealing machines of this type are available from Ro-An Industries Corp of Middle Village, NY. The Ro-An Industries style bag sealing machine 1050 is illustrated where the web is intermittently positioned into a tooling station. Roll 1030 positions the non-woven loop material 1008 with hook strip 1010 and weld bead 1014 into the bag sealing machine such that fold 1062 is laid on top of weld bead 1014, and then the weld bead 1014 and fold 1062 are positioned under weld sealing station 1066. Weld sealing station 1066 permanently joins folded plastic sheet 1054 and composite material 1007 using weld bead 1014.

Moving towards the left, heated seal bar 1068 simultaneously seals edges of bag 1006 using radiant heat and cuts the web in the cross machine direction against anvil roller 1070. In the process illustrated in FIG. 7, the web is intermittently advanced, so that at each advance, there is a period within the process where the device 1068 acts on the web. The repeat length W₁ defines the width W₁ of the strap and is determined by the stroke of the film advance. Particularly wide sheets 1007 may be longitudinally folded for processing through equipment.

Another example of flat bag-sealing machines is available from GN Packaging Industries of Mississauga, Ontario, Canada. Using the GN style of bag sealing machine, wrap-bag 1000 can be manufactured in a similar way to the Ro-An style bag sealing machine except that the cross machine edge of bag 1006 is sealed first by a weld sealing station and then an unheated knife edge cuts the edge against a flat anvil.

In another example of the process illustrated in FIG. 7, the rib 1002 and groove 1004 fasteners are engaged together and then positioned in between the two folded sides of plastic film 1054 as plastic film 1054 is center folded upon entering the machine 1050. This can be done by continuously sliding an engaged rib 1002 and groove 1004 fastener strip into rollers 1060 in between the two folded sides of plastic film 1054. Moving to the left, the engaged rib 1002 and groove 1004 fasteners are welded to the two folded sides of plastic film 1054 using top and bottom heat sealers. In a related example, the plastic film 1054 can be manufactured as a tube with one end slit to form a pre-folded plastic film. In this example, the two edges of the plastic film 1054 are separated to enable entry of the engaged rib 1002 and groove 1004 fasteners.

In still another example, hook and loop fasteners can replace the rib 1002 and groove 1004 fasteners to make another type of wrap-bag. Such a wrap-bag can be manufactured by welding on a hook strip and a non-woven loop strip to opposite edges of plastic film 1054 prior to entering the machine 1050. In this example, bag 1006, closed with hooks and loops, is not necessarily water tight but for certain applications there is no problem with some leakage of water from the bag.

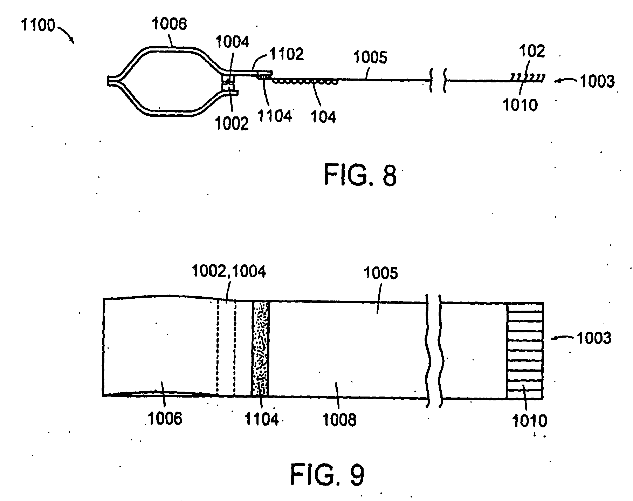

FIGs. 8 and 9 illustrate another wrap-bag 1100. In this variation, bag 1006 is formed using a j-fold such that a lip 1102 extends past groove 1004 and the opening of bag 1006. In some applications, it is desirable that the edge of the bag 1006 not directly connected to the strap be placed directly against the object about which the bag is wrapped. A weld 1104 permanently joins composite material 1007 in the form of a strap 1005 to lip 1102.

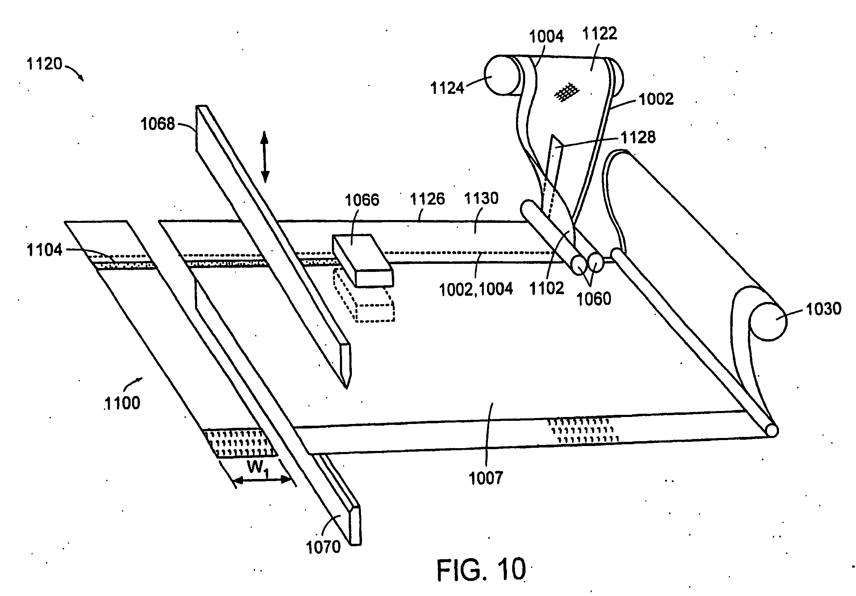

Similar to wrap-bag 1000, wrap-bag 1100 can also be made with a folded plastic sheet having continuous rib and groove fastener strips in a continuous process using flat bag making machinery. FIG. 10 illustrates a machine 1120 and method for making the cold pack wrap-bag 1100 described above. Plastic film 1122 is supplied as a roll 1124 with rib 1002 and groove 1004 fasteners previously welded to or integrally formed with the film 1122. Roll 1124 of continuous plastic film 1122 is positioned such that a fold is managed at aj-fold 1126. A folding bar or board 1128 is provided. Rollers 1060 pinch the j-fold 1126 just after the fold plate or folding board 1128 creates the j-fold 1126. Folded sheet 1130 enters into a flat bag sealing machine on top of non-woven loop material 1008 with hook strip 1010 and weld bead 1014 to form the wrap-bag 1100. Roll 1030 positions the non-woven loop material 1008 with hook strip 1010 and weld bead 1014 into the bag sealing machine such that lip 1102 is laid on top of weld bead 1014, and then the weld bead 1014 and lip 1102 are positioned under weld sealing station 1066. Weld sealing station 1066 permanently joins folded plastic sheet 1130 and composite material 1007 using weld bead 1014.

Moving towards the left, upper heated seal bar 1068 again simultaneously seals edges of bag 1006 using radiant heat and cuts the web against lower anvil roller 1070. In the process illustrated in FIG. 10, the web is intermittently advanced, so that at each advance, there is a period within the process where the device 1068 acts on the web. The repeat length W₁ defines the width W₁ of the strap and is determined by the stroke of the film advance.

The variations of the process of FIG. 7 described above similarly apply to the process of FIG. 10. Thus, a GN style machine can be substituted for the machine illustrated in FIG. 10. Furthermore, the rib 1002 and groove 1004 fasteners can be welded in-line using machine 1120. Similarly, hook and loop fasteners can be substituted for the rib 1002 and groove 1004 fasteners for a non-water tight bag.

In another variation of the wrap-bag 1100 of FIG. 8, the wrap-bag 1100 can be made without the rib 1002 and groove 1004 fasteners, such that bag 1006 has an open end at the lip 1102. In this variation, the contents of bag 1006 are kept inside bag 1006 by the engagement of hooks 102 with the loops of material 1008 when wrap-bag 1100 is wrapped around an object.

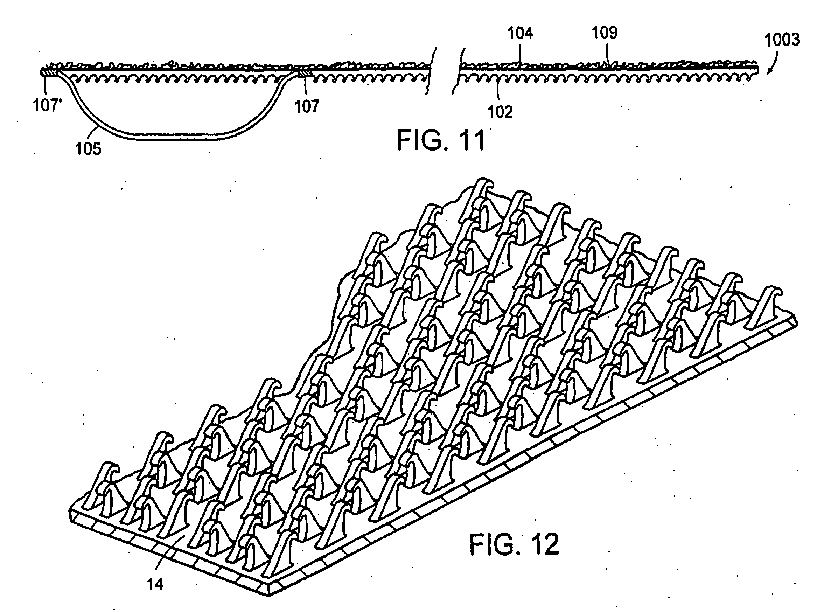

Another wrap-bag 100 is illustrated in FIG. 11. Similar to the above-described embodiments, the extended strap portion of the wrap-bag is a flexible, self-securing strap 109 extending to a free distal end 1003 and having broad fields of engageable hook and loop fasteners 102 and 104, respectively. The field of loop fasteners 104 disposed on one broad surface is arranged to engage the field of hooks 102 on the other broad surface.

Such products are conveniently manufactured by uniting a preformed web of loop material with a running length or lengths of plastic hooks, or hook preforms that are subsequently finished into loop-engageable hooks. Appropriate welds are formed as required for a given application, and the continuous material is cut at a selected repeat length, either to complete the bag-wrap, or to complete a subassembly of it.

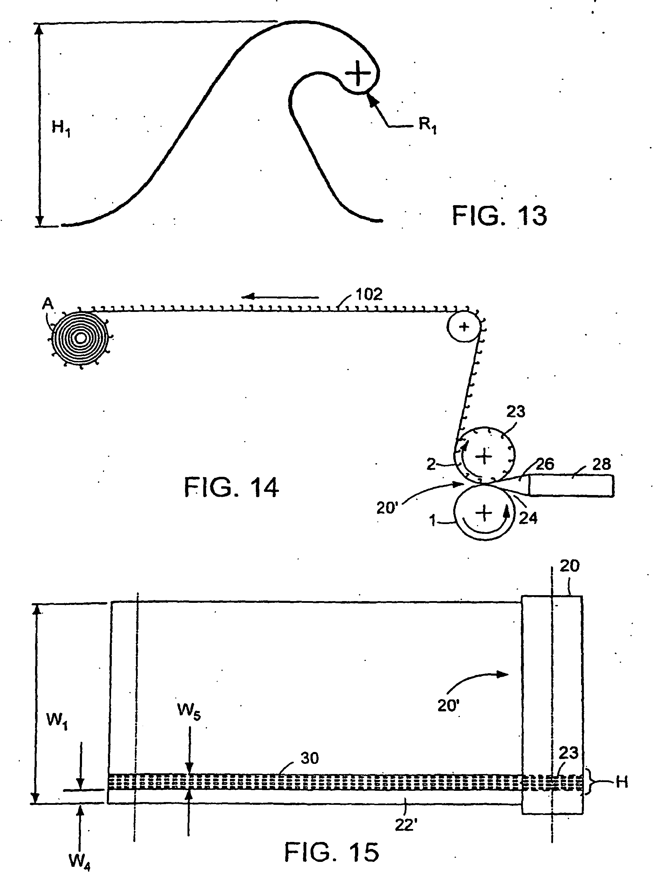

Referring to FIG. 12, the hook fasteners 102 may be of molded form available from Velcro, USA under designation CFM29, shown magnified in FIG. 13. Its dimensions are H₁ of 0.0378 cm (0.0149 inch) and R₁ of 0.00381 cm (0.0015 inch). As a specific example, the loop material may for instance be non-woven hook-engageable material, available from Velcro, USA as loop L3310, formed according to techniques shown in U.S. Patent 6,342,285, the full content of which is hereby incorporated by reference. In other cases of hook and loop construction, other low-cost hook forms and loop materials may be employed, for instance hooks formed by post-forming molded stems and loops formed by light weight, inexpensive knit materials, for instance knitted loop material having a weight of less than 4 ounces per square yard, preferably less than 2 ounces per square yard.

In the example illustrated in FIGs. 14 and 15, a flat plastic sheet 22' of width W₁ is produced by calender action upon formable resin extruded by flat die 26 from moldable resin provided by extruder 28. In this case no preformed material is introduced to the forming nip 20'. The upper roll 2' of the calender nip 20', in a widthwise defined region H, has mold cavities 23 in its surface that define loop engageable hooks, stems or other hook preforms, self-engaging formations, or other fastener features. In the illustrated embodiment, loop-engageable hooks 102 of form shown in FIGs. 12 and 13 are molded at hook section 30 located distance W₄, for example 3.175 cm (1.25 inches), from the edge of the material. In this example the hook band 30 is of width W₅, for example 3.81 cm (1.5 inches). This process, with fixed mold cavities, can produce the loop-engageable hooks such of FIGs. 12 and 13 or hook preforms of a selected desired shape or shapes suitable for post-forming action, etc. Molding occurs as the calender stack produces the plastic sheet. A completed composite material from which wrap-bags may be formed is completed by joining a preformed band of loop material to an appropriately selected section of the plastic sheet. Heat sealing, adhesive, or other joining processes may be employed, dependent upon the material and construction of the loop material and the required quality of the joint. For instance, if a binder material in the back of a preformed loop material is an acrylic resin, a heat seal weld may be formed to the sheet 22' along marginal edges, or mid bands of the loop material, by heat sealing action with a compatible plastic of the carrier sheet 22'. For instance, sheet 22' may be of polyethylene. In other cases, loop or other fastener material may be formed in place upon the resin sheet 22' after the latter is formed.

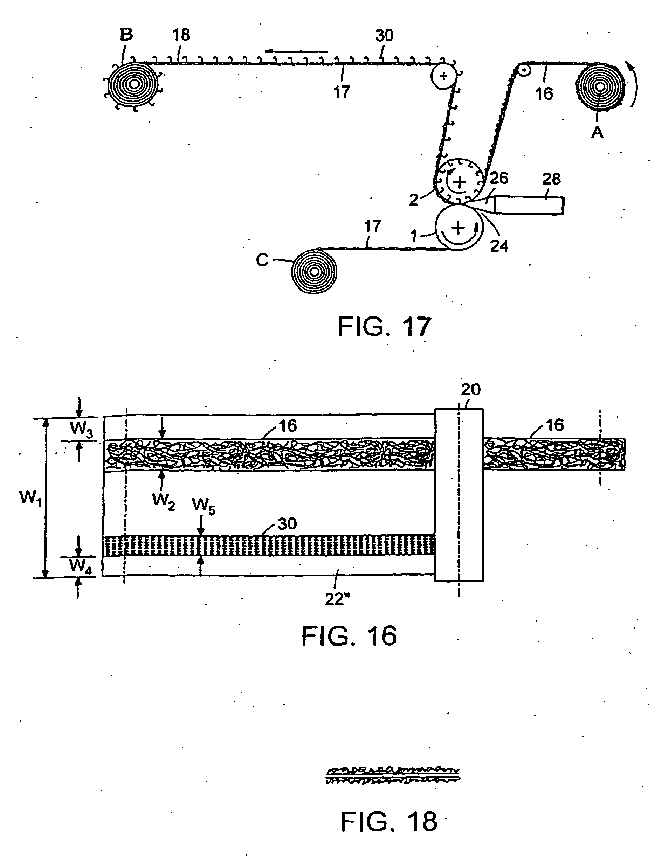

In the example of FIGs. 16 and 17, both hook and loop fastener components are joined *in situ* to a plastic sheet 22" being produced by calendering action. Preformed hook-engageable loop material 16 is introduced into the calender nip while a band 30 of loop engageable hooks is molded *in situ* to extend integrally from a surface of the resin film as described with respect to FIG. 14. As shown by FIG. 16, the web exiting the process has respective continuous machine-direction bands of hook fastener and loop fastener components at appropriate locations on the plastic carrier sheet, all components having been united *in situ* by the sheet-forming and joining calender process.

The general concept of *in situ* lamination while molding hooks is explained in U.S. 5,260,015 by Kennedy et al., and *in situ* lamination of strips of molded hooks, per se, is disclosed in U.S. 6,205,623 by Shepard et al., the contents of both of which are hereby incorporated by reference.

In the example of FIG. 16, the bands of hooks or hook preforms and loops are shown disposed on the same surface of the web 22". However, by alternatively, or simultaneously having a continuously supplied loop material following the path from takeoff roll C on the opposite side of the incoming resin, the band of loop material is introduced to the lower roll on the bottom side of the plastic sheet so that a band or bands of hooks or hook preforms and a band or bands of loops are disposed on opposite sides of the formed web. A composite formed with the full arrangement of FIG. 17 forms the material shown in FIG. 18.

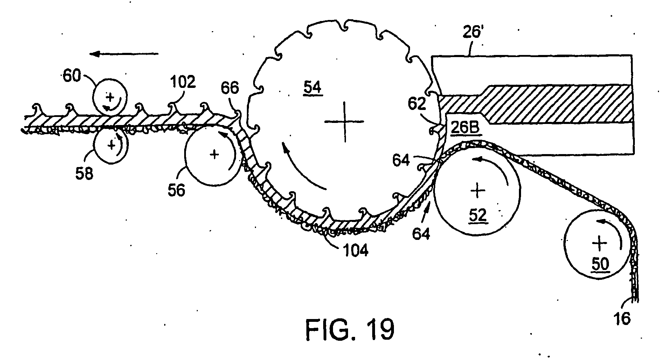

The apparatus and process illustrated in FIG. 19 can alternatively be employed. In this case the plastic sheet 22"', and a section with hooks 102 extending from the plastic sheet is formed by mold roll 54 having a band of hook cavities, as the plastic from the extruder passes through a gap formed between the roll and a complementary-shaped extension 26b of the extrusion die 26'. While the resin is still molten, the loop material is introduced and laminated to the resin at a nip 64 formed between the mold roll 54 and pressure application roll 52. At this point, hooks 66 are still in their mold cavities, protected from the effects of laminating pressure.

In these and other roll-forming arrangements, provisions may be included to impart cross-machine strength to the formed composite web. In some cases this is provided by a cross-machine-strong preformed fastener material or its carrier. In other cases, a reinforcing scrim may be introduced to the roll-forming station in a manner by which the scrim is embedded in the sheet being formed. Examples are introduction of an open reinforcing scrim on the molding roll side of resin entering the forming gap through which the resin passes in entering the mold cavities, and coextruding two layers of resin while interposing a running length of the scrim between the layers before the layers enter the forming gap. The coextruded resin may be of the same or compatible materials. In one case a relatively stiff resin is employed to form the hooks and a thin upper part of the base layer, and a compatible resin, for instance a copolymer or blend, having elastomeric properties may form the predominate thickness of the base layer under the hooks and a calendered sheet extension as well. In this manner a wrapping material with elastic properties is formed. In some cases the reinforcing material may be omitted. In other cases the predominate thickness of the base layer may instead be selected for its toughness and the reinforcing layer may be omitted.

In a further example, the basic apparatus and process described with respect to FIGs. 16 and 17 is employed in FIGs. 20 and 21, however using wider loop material and a wider mold cavity section in the mold roll. Two parallel fastener bands, of loop-engageable hook 102, and hook-engageable loop 104, respectively, are formed on the same side of an *in situ* molded, machine-wide, plastic carrier sheet 111. Resin is introduced into the nip of the calender stack over the full roll width, and longitudinal, inter-engageable rib and groove rails 1002, 1004 are simultaneously molded in the back face of the resin sheet in corresponding molding grooves (not shown) defined in the pressure roll 1. The preformed loop material 16 from supply roll B is of appropriate width and position to leave, at the adjacent edge, a weld flange 114 free of loop material. The width H₂ of the mold section of roll 2 which carries mold cavities 23 is also sized slightly less than half of the width of plastic sheet 111. This produces a wide band of hooks 102 or hook preforms that is bordered at the outside edge by weld flange 112 of calender-produced plastic sheet.

Referring also to FIGs. 23, 24 and 25, a narrow center region 100 between the bands of hook and loop is also devoid of hooks and of loop material. A central machine-direction fold axis A is defined by suitable formation of the surface of roll 2 to facilitate folding the laminate crosswise to the machine direction. For example, a small, circumferential central raised formation F (FIG. 23) on the surface of roll 2 forms in the plastic sheet a machine direction region or notch N of decreased thickness t_{d}, about which the plastic sheet will preferentially fold in creating a continuous composite material for forming wrap-bags. In some preferred cases, weld bands of plain resin lie along each side of the fold line axis A, to enable welding of the two plastic layers together in this region after folding. Following molding and *in situ* laminating, the wrapping material is cooled, removed form the mold roll, passed over tension roll 120 and rolled up into supply roll 130.

Thus, a wide roll-formed sheet 111 having hook and loop bands 102 and 104 of substantially half-width extent of the sheet 111, integral outer weld flanges 112, 114 of width W₆ of about 1.27 cm (0.5 inch), and in some cases an integral rib-and-groove closure, can be produced for forming wrap-bags and other products. The material is produced by pressure action by a roll, preferably the calender action described. This material is suitable for forming one or more carrier pockets or pouches 119 (FIG. 26) and the flexible strap 125 of a wrap-bag.

As suggested in FIG. 25, 26 and 28, as this material is folded about middle machine direction fold axis A, the weld flange sections meet to form one edge of the composite material, with the rib and groove formations aligned for engagement. Other weld lines define the ends of the discrete length of composite material and the location of a pouch to be formed, to create a flattened tube covered with loop-engageable hooks 102 and hook-engageable loops 104 on the oppositely directed sides. In FIG. 25, lines W transverse to axis A define regions where transverse welds may be formed. FIG. 26 illustrates the welded wrapping unit. Welding flange sections 112', 114' of FIG. 25 form weld 115 and flange sections 112", 114" form weld 113, each parallel to axis A. Transverse end weld portions 122, 123 and internal pouch-defining weld 124 complete the unit.

Preferably, a weld flange along one or more pouches being formed is left free to provide a top pouch opening or openings for access by the user, as shown in FIG. 26, exposing the mated rib and groove formations that then serve as a recloseable closure for the pouch. The continuous web that has been folded in half about axis A is advantageously transversely welded with double width at 121 in an in-line process, at a selected repeat length to define the length of the flexible wrapping unit. After this, the running length of material is cut at the repeat length, to form weld regions 122 and 123.

As another option, plain weld flange sections 112 and 114, illustrated in FIG. 24, may be omitted and the fields of hooks and loops allowed to extend to the edge of the composite material. In this case, welds 113 and 115 are formed by application of heat and pressure through the hook and loops to cause the corresponding portions of the plastic backing to weld together.

As mentioned, transverse welds at the ends, 122 and 123 (typically sections of a double wide single weld 121 which is cut to sever the leading unit during production) define the repeat length for the continuous production process. As desired, a selected number of intermediate transverse welds 124 are applied through the thickness of the hook and loop sections of the composite to form one or more pouches of limited dimension along the material, or to provide optional cut lines at which the user may choose to shorten the material by cutting. In the example of FIG. 26, one intermediate weld 124 defines with end weld 123, pouch 119.

By making the film of the *in situ* laminate of resin capable of strong continuous heat seals, such as polyethylene, the flanges can be heat-sealed to each other to provide water tightness and strength along the edges as well as along the pouch walls. This ensures containment of the chemical reactants. An advantageous resin for such embodiments is commercially available linear, low-density polyethylene such as LL-6407 Exxon Mobile resin.

The composite material may be configured to include closing flaps (not shown) adjacent the pouch opening, to be folded over to releasably cover the opening of the pouch.

Referring to FIGs. 28 and 29, wrap-bag 101 securely holds the contents of the pouch 119 close to an object 128. The flexible extension of the bag (i.e., the portion of the wrap extending from the pouch 119), fastens to itself by touching the loop material 104 to the hook surface 102, as illustrated in FIG. 29. Because of the weld 122 provided at the free end of the wrap shown in FIG. 26, a small dead region D of fastener material is provided that is incapable of fastening engagement (see also FIG. 27). This provides an easy peel, free standing tip to grasp to initiate unwrapping. Thus, while the entire length of excess wrapping tightly engages itself without an undesirable loose tail, the extreme tip D remains free to be grasped.

Selection of a suitable preformed non-woven material depends upon cost, quality and number of uses objectives, e.g. whether it is to be a single use device or whether a number of repeated uses are desired. For economy, a non-woven product formed by needling staple fibers followed by stretching and binding as shown in U.S. Patent 6,342,285 is useful. This material is available from Velcro, USA as loop L3310. To enable a large number of repeated openings and closing, the loop material may be a knit fabric with acrylic binder at its back such as Loop 3905 available from Velcro, USA. For intermediate cost applications, extremely light weight knitted materials may be employed.

For low cost synthetic resin for the hooks, good weldability, good sealing qualities, etc., polyethylene is advantageous, while for certain performance characteristics, other resins are selected, e.g. PVC for comfort, conformability, or RF welding; polypropylene for strength and cost; and polyester for high strength applications where a degree of stiffness of the bag wrap extension is useful.

In another embodiment having extensive loop coverage and breathability, the plastic sheet side of the laminate in the loop area is not entirely covered with resin sheet. Instead, parallel, spaced apart bands of resin are provided between which are bands of porous loop material, free of resin. The free regions can provide porosity that enables air or moisture to pass through the strap of the wrap-bag. Two major plastic bands, for instance, may be provided on the porous material in such an embodiment, at the top and bottom long edges of the strap, to enable major welds to be strategically placed for holding a folded assembly together. Between those weld regions, only two or three narrow beads or bands of resin may be employed, e.g. bands 0.3175 cm (1/8 inch wide), that leave most of the area free to breathe. Such beads or bands of resin enable formation of spot welds sufficient to define a pouch capable of retaining an inserted pack or device. During formation of the wrap-bag, these beads or bands of resin, carried on the inside surface of the porous material, are engaged by a transversely extending weld bar to form spot welds to matching, weldable portions on the opposite side of the folded material. Where the opposite side comprises a continuous plastic base layer, welding of a portion of each bead is assured wherever a cross-wise extending linear heated weld bar may engage the plastic bead against the opposed plastic surface. In such cases, little care is needed to provide registry.

Referring now to FIG. 30, two preform materials 360, 362 can be joined to form the wrap-bag shown in FIGS. 31 and 32. Component 360 comprises a loop band 300 *in situ* laminated to a calendered layer of resin 374. Only a small margin of the back of the loop material overlaps the calendered resin 374 band, and the latter extends beyond the loop material, used to form one side of a pouch of width R. The material is cut transversely to machine direction MD so that the length of web 360, W₁ extends in the cross-machine direction, in relation to the starting material. Web 362 is created using the apparatus and process describe in respect of FIGs. 14 and 15 and again is cut transversely, in the manner that its length W₁ is transverse to machine direction MD. Web 362, of roll-formed resin, has a hook strip 364, and a sheet-form resin flange 366 lying outwardly beyond the hook strip. The remainder of web 362 is calendered resin sheet 368, if length to form the other side if the pocket and a section of the body of the wrapping. The two components.360, 362 are joined together, each component, as joined, having an end overlapped by the other component and an opposite free end, with the hook section 364 facing to one side and the loop 300 facing to the other side. Transverse welds 278, 380 are located at respective ends of each of the overlapping component, the amount of overlap thus determining the width R of the pouch 119"'. The resulting wrap-bag has an overall length W₃. FIG. 33 shows the joined webs having a bottom weld 382 forming the bottom of the pouch 119"' and the top of the pouch left open. The pouch defines an internal compartment 384 for receiving objects. Given a typical calender stack with width W₁ of about 60.96 cm (24 inches), a pouch width of about 10.16 cm (four inches), and welds 380, 378 of width about 1.27 cm (0.5 inch), then W₃ can be 109.22 cm (43 inches) when using the full width capability of the calender stack to form the two components 360, 362. Wider machines can form correspondingly longer products.

In the embodiment illustrated in FIG. 34, weld 378 is not created while a top weld 386 is created, extending in the direction of the length, across the top of the overlap of sheets 374 and 368. Thus, the pouch 119"' is open from the side, side opening 379, and closed from the top. This allows objects to be placed in the pouch from the side. When the wrap-bag is wrapped around an object with loop surface 300 on the inside, the bag strap covers the side opening 379 to prevent the pouch contents from slipping out.

In some cases, a non-woven material with acrylic binder having an elastomeric characteristic in the cross-machine direction is employed as a stretchy loop material 300. Such a material and other stretchy loop materials are described in PCT/US01/08100, published on September 20, 2001, which is hereby incorporated by reference on its entirety. In such a wrap-bag the strap portion of the bag is stretchy in the direction of the length W₃ (i.e., in the cross-machine direction only during the prior *in situ* lamination step in which the composite was formed). The elastic stretchiness achieved in the direction of the length of the wrap enables the user to tighten the wrap and fasten it, to permit freer motion of the wrapped object while ensuring good contact between the wrap-bag and the wrapped object.

In some embodiments, the base fabric of the loop material includes thermoplastic material, such as thermal adhesive fibers or thermoplastic binder distributed through the thickness of the material. This renders the loop material capable of being heat-sealed to itself, while preserving its porous character in regions beyond the heat seal. In such cases, the loop material is not backed with a roll-formed sheet layer, so that the wrapping may be air-permeable.

The wrap-bag 601 of FIGs. 35-37, including strap 609, is formed by mating a running length of hook sheet 606 with a resin film-backed loop material 600 (loops facing up) at a region of overlap with a first machine direction weld 602, and then welding a single sheet of pre-formed biaxially-oriented film 604 at a second machine direction weld 608 at the outer edge of the hook material 606. A pouch 618 is then formed between the overlying film 604 and the back of the hook material 606 by applying welds at two sides, leaving one side 616 open. In the case shown, side welds 610, 612 are both made in cross machine direction leaving the pouch opening 616 at the interior of the wrapping.

Wrap-bag 601 may manufactured entirely on bag-making equipment well-known in the packaging industry. Referring to FIGS. 38-39A, three continuous sheets 600, 606, and 604 are brought together joined using reciprocating heat seal jaws. From left to right are seen a roll of continuous hook material 640, a roll of continuous loop material 641, and a reciprocating weld head 643 for weld 602. Following is a film roll 642 for the top layer of film 604 and a secondary weld head 644 for weld 608. Weld 608 joins the film 604 to the end of the backside of the hook sheet 606. A cutoff jaw 647 slices individual wrap-bags from the continuous web and separates welds 610 and 612 of two individual wrap-bags. The cut through the welds 610 and 612 of the continuous sheet allows weld 612 to stay on the trailing edge of one finished wrap-bag, and the weld 610 stays on the leading edge of another wrap-bag.

FIG. 39 shows the web after creation of the first weld 602. FIG. 39 shows the relationship between hook strip 606 and loop strip 600. FIG. 39A shows the web after the second weld head 644, illustrating the relationship of the hook and loop, and the addition of film web 604 and weld 602.

Typical biaxially-oriented films for preformed sheet 604 are polyethylene or polypropylene based, polyethylene being the least expensive and most economical. A preferred embodiment is a hook sheet 606 made of polyethylene resin, either a linear low standard injection mold grade or film grade.

The manufacturing process may be performed on a bagging machine with a limited capacity to handle the joined sheets 601 widthwise. Folding the non-woven loop sheet 600 throughout the process enables the processing of a much longer wrapping on a machine that typically would not handle such a long item. Typical bagging machines are about 137.16 cm (54 inches) in width, so wrap-bags longer than 101.6 cm (40 inches) may require folding to process. In this case, FIG. 40 shows wrapping 601 as it finishes the manufacturing process of FIG. 38, with its loop section 600 folded (as part 648) so that the wrapping can fit widthwise through the manufacturing process. This fold is accomplished during the process of FIG. 38 by continuously folding sheet 600 as it is unrolled from supply roll 641.

In another embodiment illustrated in FIGs. 41 and 44, the main body of a wrap-bag 708, as well as the entire pouch and strap 709, are formed of preformed loop material, and the hook material is joined in the region in which the loop material is folded back upon itself. Again, two welds, both transverse to the machine direction, form the sides of the pouch and the pouch opening is inside the wrap-bag. A sheet 702 of hook-engageable non-woven loop fabric of length L₂, greater than L₁, is folded along an axis 704 transverse to its length forming sections 700 and 706 as illustrated in FIGs. 41 and 43. This fold 720 is done in such a manner that the folded sheet 703 has hook-engageable loop sides of 700 and 706 facing one another. A sheet 718 of hook web of length L₄, a minor (e.g. less than 1/4) part of the overall length L₁ of the wrap-bag, is also provided. This hook sheet 718 can be manufactured using the process illustrated in FIGs. 14 and 15. Hook sheet 718 is joined to the end of sheet 703 at weld 710 with hooks facing the opposite direction of loops of section 706 as illustrated in FIG. 44. Welds 712 and 714 seal loop section 706 to loop section 700 forming a pouch 724 with opening 722. The opening 722 is oriented such that whenever the bag-wrap is securely wrapped about an object, the contents cannot fall out because the opening 722 is against the wrapped object.

A preferred embodiment uses polyethylene resin for the hook material 718, but other resins that are compatible with the non-woven loop material to make the pocket and the wrap may also be used. The hook portion 718 may be oriented such that when the weld 710 is created, the hooks melt to form a good attachment without need for film on the back of the non-woven loop strap 706.

Another wrap-bag construction 742 is illustrated in FIGs. 45 and 46. In this example the pouch opening is resealable with hook and loop touch fastener strips. One side portion 746 of the pouch is a preformed assembly consisting of a composite loop material strip 750 and a preformed biaxially-oriented film sheet 744 welded together at weld 748. The preformed biaxially-oriented film sheet 744 is joined to section 758 at weld 754 to form pouch 756. Hook section 762 functions both as one side of the pouch closure and for engaging the fibers of the opposite side of the wrap to secure the wrap-bag in place. The sides of the pouch are formed by welds 764 that are created as the individual wrap-bags are severed from the web.

Another wrap-bag that includes biaxially-oriented plastic sheet to form the pouch is illustrated in FIGs. 47-49. The pouch may be formed by folding a preformed biaxially-oriented plastic sheet 811 or by joining two separate flat sheets at a weld 813. A stretch component 810 on the hook end enables the wrap-bag to be tightly fastened. Non-woven loop sheet 814 is joined to folded film 811 at weld 812. Non-woven loop sheet 814 either has a polyethylene backing that is applied prior to this assembly, or it can be non-woven loop material without polyethylene backing, depending on the required quality of the weld and the other requirements of the application. The amount of hook and loop material in both of these cases can be adjusted according to cost concerns. The opening of the pouch can be made sealable by adding a pressure-sensitive assembly 816 to the film 811 prior to folding the film 811. The pressure sensitive assembly 816 consists of a pressure-sensitive adhesive strip 817 with a release tab of 818 and that allows the pouch to be sealed after the pack is manually inserted into the pouch. After release tab 818 is pulled off, pressure sensitive strip 817 sticks to the other side of folded film 811 and seals the pouch opening shut. This embodiment may also be varied by replacing the film 811 with a woven or non-woven knit. This replacement material may be waterproof and is commercially available as Tyvek®, Typar®, or scrim.
The wrap-bag 877 illustrated in FIGs. 50 and 51 can be formed with a pouch 876 directly from the calender roll process. Since the calender roll process described above laminates the non-woven loop material 875 with a plastic backing of hooks 874, preventing this lamination in a select area creates a pouch 876. This is accomplished by printing an overprint varnish 879 on the non-woven loop material 875 before the calender process which prevents lamination in certain areas between the hook backing 874 and the non-woven loop material 875, to form a three-sided pouch 876 with an open edge and strap 889.

In one example of usage of bag strap 1200, referring to FIG. 52A, wrap-bag 1200 is wrapped around a chassis 1220 and bag 1006 to hold loose components 1222 associated with the chassis 1220. Parts 1222 can include nuts, bolts and brackets, for example. Thus, prior to final assembly of the components 1222 to chassis 1220, no adhesive is required to attach parts 1222 to chassis 1220, so damage to paint on chassis 1220 by removing tape is avoided.

The wrap-bag can also provide a reliable means of holding detonation charges in place against a target structure. Referring to FIG. 52B, wrap-bag 1200 can be applied about a cement or other structural column 1240 for positioning a shaped explosive charge 1242 for demolition. In military applications, cement column 1240 might support a bridge or a building to be demolished. In civilian applications, cement column 1240 supports a building or other structure to be demolished. In this example, explosive charge 1242 has wires 1244 attached to battery 1246 for exploding cement column 1240. Wires 1244 are attached to electrodes 1248. Electrodes 1248 have sharp pointed ends capable of piercing through thin fabric and plastic materials. For this usage, a user inserts explosive charge 1242 inside bag 1006 and then attaches explosive charge 1242 to cement column 1240 by wrapping bag strap 1009 around the cement column. Subsequently, the user can insert electrodes 1248 through material 1008 and 1054 into bag 1006. Unlike some adhesives, the hook-and-loop fasteners of the strap can reliably hold the charge in place, even after several days and extreme changes in temperature.

Other uses of wrap-bag 1200 wrapped about cement column 1240 include holding, forming and accelerating setting of repair materials or applying to a post or structural joint or to heat the joint during curing.

Another wrap-bag 1300, shown in FIG. 53D, can be readily manufactured in a continuous process using the processes and apparatus disclosed in pending U.S. patent application 09/808,395, filed March 14, 2001. Referring first to FIG. 53A, three separate substrates are simultaneously fed into a molding nip. The substrates, shown here in transverse cross-section (i.e., looking in the direction of material flow) include a light knit material 1302, a sheet of film 1304, such as a polyptopylene or polyethylene film, or a paper-backed film, and a strip of non-woven loop material 1306. In the nip, two discrete bands 1010 of fastener elements 102 (or fastener element preforms) are molded *in-situ* along the film sheet 1304 and the knit material 1302, respectively (FIG. 53B). In the same nip, the film sheet is permanently bonded to the knit material along a center region 1308, and the non-woven loop strip 1306 is bonded to the film sheet 1304, such as by heat-staking. Such heat-staking methods are disclosed in U.S. Patent No. 6,202,260, incorporated herein by reference. The bond in center region 1308 can be created by welding the film sheet directly to the knit material, or by adding a molten strip of resin over a series of perforations, for example, in the film sheet 1304, the latter method being particularly useful when the material of sheet 1304 and knit 1302 are weld-incompatible, and/or when sheet 1304 is of paper or paper-backed film. As the non-woven loop strip 1306 is bonded to film sheet 1304, sufficient heat and pressure may be applied to also bond the back side of sheet 1304, underlying loop strip 1306, to the upper surface of knit 1302. Alternatively, the back surface of sheet 1304 can be coated with a weld-inhibiting material, such as an overprint varnish, in the area underlying the loop strip, so as to inhibit bonding of sheet 1304 to sheet 1302 as the loop strip is staked, such that the loop-edge of sheet 1304 remains free as shown. In cases where the loop-edge of sheet 1304 is left free of knit 1302, the hook-edge of sheet 1304 may, if desired, be bonded to knit 1302 during the formation of hook strips 1010, such as by providing a line of perforations through sheet 1304 where the inner hook strip will be formed. In any event, at least one half of sheet 1304 should remain free of knit 1302 as the product exits the molding nip.

After exiting the nip, the fastener elements (if preforms) may be headed to form loop-engageable heads, such as by passing them under a forming roller. The hook-half of sheet 1304 is then folded over at center region 1308 to engage the inner hook band 1010 with the non-woven loop strip 1306, to form the continuous preform from which the bag bodies will be formed. It should be noted that sheet 1304 may be folded to create a pleat (not shown) adjacent bonded region 1308 to form bag bodies with expandable gussets opposite their openings, if desired.

The continuous sheet product (now as shown in FIG. 53C) is then cut transversely to form individual wrap-bags 1300, as shown in FIG. 53D, with sheet 1304 forming the opposing sides of a reclosable bag 1310 secured at its bottom edge to a strap 1312 formed by knit 1302. The transverse cuts can be performed so as to seal the lateral edges of the bag, and to secure the lateral bag edges to the edges of the strap, if desired. Alternatively, the transverse cutting may simply cut sheet 1304 to form free edges that are later sealed together as separated from the knit substrate of the strap, such that the bag body is completely free from the strap except along its lower edge, as shown.

The process described above may be readily modified. For example, one may start with either sheets 1304 and 1302, or sheets 1304 and 1306, pre-joined before entering the nip. The bands 1010 of fastener elements may be preformed and bonded to the substrates by welding or adhesives, rather than bonded during molding. Knit 1302 may be replaced with a sheet of paper, film or other flexible sheet substance, to which a field of loop material is bonded to a side opposite the fastener elements.

A number of embodiments of the invention have been described. Nevertheless, it will be understood that various modifications may be made without departing from the spirit and scope of the invention. In addition, various other uses of wrap-bags constructed according to the above principles will be evident to those of ordinary skill. Such wrap-bags can be specifically fashioned to secure samples and specimens under difficult working conditions, such as in space or underwater, for example. Bag-wraps can be fashioned for wrapping about a body part, such as to form a pocket or concealed pouch, for carrying small or paper items, such as fishing licenses, money or passports. The bag may also be used to strap monitors to a patient, such as a fetal monitor during delivery, or a heart rate monitor under pressure against the skin. Many other uses will be apparent.

## Claims

1. A bag (1000,1100,100,101,601,708,742,877,1200,1300) comprising
a bag body (1006,1310,119,119"',618,724,756,876) defining therein a compartment (384) accessible through an opening (379,616,722) at one end of the bag body (1006,1310,119,119"',618,724,756,876); and
secured to the bag body (1006,1310,119,119"',618,724,756,876) along one edge thereof and extending therefrom to a free distal end (1003), a flexible strap (1009, 1005, 109, 125, 609, 709, 706, 889, 1312) of width sufficient to span at least a majority of the compartment (384), the strap carrying an array of fastener elements (102) on an opposite side thereof, each fastener element (102) having a stem extending integrally from a band of resin extending across the strap (1009,1005,109,125,609,709,706,889,1312);
the strap (1009,1005,109,125,609,709,706,889,1312) being of sufficient length to wrap about an object, with the strap (1009,1005,109,125,609,709,706,889,1312) overlapping the bag body (1006, 1310, 119, 119"', 618, 724, 756, 876) to engage exposed fibers of the bag (1000,1100,100,101,601,708,742,877,1200,1300) with the fastener elements (102), to secure the bag to the object.

2. The bag (1000,1100,100,101,601,708,742,877,1200,1300) of claim 1 wherein the band of resin encapsulates surface features of the strap (1009,1005,109,125,609,709,706,889,1312) to form an inseparable laminate.

3. The bag (1000,1100,100,101,601,708;742,877,1200,1300) of claim 1 wherein the band of resin is an integral region of the strap (1009,1005,109,125,609,709,706,889,1312), the strap being a unitary sheet of the resin and extending beyond the array of fastener elements.

4. The bag (1000,1100,100,101,601,708,742,877,1200,1300) of any of the above claims wherein the strap (1009,1005,109,125,609,709,706,889,1312)
extends beyond the edge of the bag body (1006,1310,119,119"',618,724,756,876) a distance greater than about twice a width of the bag body (1006,1310,119,119"',618,724,756,876) measured from the same edge in opposite direction.

5. The bag (1000,1100,100,101,601,708,742,877,1200,1300) of any of the above claims wherein the strap (1009,1005,109,125,609,709,706,889,1312) consists essentially of a sheet of loop material with the band of resin extending thereacross.

6. The bag (1000,1100,100,101,601,708,742,877,1200,1300) of any of the above claims wherein the bag body (1006,1310,119,119"',618,724,756,876) is secured to the strap (1009,1005,109,125,609,709,706,889,1312) along multiple edges of the bag body.

7. The bag (1000,1100,100,101,601,708,742,877,1200,1300) of any of the above claims wherein the strap (1009, 1005, 109, 125, 609, 709, 706, 889, 1312) is an integral extension of one side of the bag body (1006, 1310, 119, 119"', 618, 724, 756, 876).

8. The bag (1000,1100.100.101,601,708,742,877,1200,1300) of any of the above claims wherein the bag body (1006,1310,119,119"',618,724,756,876) includes a releasable closure extending along the opening (379,616,722).

9. The bag (1000,1100,100,101,601,708,742,877,1200,1300) of claim 8 wherein the closure comprises a rib-and-groove closure.

10. The bag of claim 8 wherein the closure comprises a touch fastener closure.

11. The bag (1100,601,708,742,1200,1300) of any of the above claims wherein the opening (379,616,722) faces the strap (1009,1005,109,125,609,709,706,889,1312).

12. The bag (1000,100,101,877) of any of claims 1 to 10 wherein the opening (379,616,722) extends along one side edge of the strap (1009,1005,109,125,609,709,706,889,1312).

13. The bag (1000,1100,100,101,601,708,742,877,1200,1300) of any of the above claims wherein the strap (1009,1005,109,125,609,709,706,889,1312) is resiliently stretchable in a longitudinal sense.

14. The bag (1000,1100,100,101,601,708,742,877,1200,1300) of any of the above claims wherein the fastener elements (102) are disposed in a discrete band (1010) adjacent the free end (1003) of the strap (1009,1005,109,125,609,709,706,889,1312).

15. The bag (1000,1100,100,101,601,708,742,877,1200,1300) of claim 14 wherein the discrete band (1010) is continuous and extends across the width of the strap (1009,1005,109,125,609,709,706,889,1312).

16. The bag (1000,1100,100,101,601,708,742,877,1200,1300) of claim 14 wherein the strap (1009,1005,109,125,609,709;706,889,1312) includes a graspable, non-fastening region (D) at its free end (1003), beyond the band (1010) of fastener elements (102).

17. The bag (1000,1100,100,101,601,708,742,877,1200,1300) of any of the above claims wherein the compartment (384) is defined fully within the width of the strap (1009,1005,109,125,609,709,706,889,1312), such that the strap envelops the compartment (384) when overlapped across the bag body (1006,1310,119,119"',618,724,756,876).

18. A method of releasably securing one or more relatively small components to a relatively larger object, the method comprising
placing the components in the compartment (384) of a wrap-bag (1000,1100,100,101,601,708,742,877,1200,1300) according to claim 1; and
wrapping the wrap-bag (1000,1100,100,101,601,708,742,877,1200,1300) about the object in overlapping manner, releasably securing the fastener elements (102) of the wrap-bag (1000,1100,100,101,601,708,742,877,1200,1300) to the fibers of the wrap-bag, to hold the wrap-bag to the object.

19. The method of claim 18 wherein the relatively large object is a chassis (1220), and the components (1222) are to be later assembled to the chassis (1220).

20. The method of claim 18 wherein the relatively large object is to be detonated, and the components comprise one or more explosive charges.

## Patentansprüche

1. Beutel, (1000, 1100, 100, 101, 601, 708, 742, 877, 1200, 1300), umfassend
einen Beutelkörper (1006, 1310, 119, 119"', 618, 724, 756, 876), der darin ein Abteil (384) definiert, das durch eine Öffnung (379, 616, 722) an einem Ende des Beutelkörpers (1006, 1310, 119, 119"', 618, 724, 756, 876) zugängig ist; und
gesichert an dem Beutelkörper (1006, 1310, 119, 119"', 618, 724, 756, 876) entlang einer Kante davon und sich erstreckend davon zu einem freien distalen Ende (1003) einen flexiblen Streifen (1009, 1005, 109, 125, 609, 709, 706, 889, 1312) einer Breite, die ausreichend ist, um wenigstens einen Großteil des Abteils (384) zu überspannen, wobei der Streifen ein Feld bzw. Array von Befestigungselementen (102) auf einer gegenüberliegenden Seite davon trägt, wobei jedes Befestigungs- bzw. Festlegungselement (102) einen Stiel aufweist, der sich einstückig bzw. integral von einem Harzband erstreckt, das sich über den Streifen (1009, 1005, 109, 125, 609, 709, 706, 889, 1312) erstreckt;
wobei der Streifen (1009, 1005, 109, 125, 609, 709, 706, 889, 1312) von ausreichender Länge ist, um sich um einen Gegenstand zu wickeln, wobei der Streifen (1009, 1005, 109, 125, 609, 709, 706, 889, 1312) den Beutelkörper (1006, 1310, 119, 119"', 618, 724, 756, 876) überlappt, um freigelegte Fasern des Beutels (1000, 1100, 100, 101, 601, 708, 742, 877, 1200, 1300) mit den Festlegungselementen (102) zu ergreifen, um den Beutel an dem Gegenstand zu sichern.

2. Beutel (1000, 1100, 100, 101, 601, 708, 742, 877, 1200, 1300) nach Anspruch 1, wobei das Harzband Oberflächenmerkmale des Streifens (1009, 1005, 109, 125, 609, 709, 706, 889, 1312) verkapselt, um ein untrennbares Laminat auszubilden.

3. Beutel (1000, 1100, 100, 101, 601, 708, 742, 877, 1200, 1300) nach Anspruch 1, wobei das Harzband ein einstückiger bzw. integraler Bereich des Streifens (1009, 1005, 109, 125, 609, 709, 706, 889, 1312) ist, wobei der Streifen ein einheitliches bzw. einziges Blatt aus dem Harz ist und sich über das Feld von Festlegungselementen erstreckt.

4. Beutel (1000, 1100, 100, 101, 601, 708, 742, 877, 1200, 1300) nach einem der obigen Ansprüche, wobei sich der Streifen (1009, 1005, 109, 125, 609, 709, 706, 889, 1312) über den Rand bzw. die Kante des Beutelkörpers (1006, 1310, 119, 119"', 618, 724, 756, 876) um einen Abstand größer als etwa zweimal einer Breite des Beutelkörpers (1006, 1310, 119, 119"', 618, 724, 756, 876), gemessen von derselben Kante in entgegengesetzter Richtung erstreckt.

5. Beutel (1000, 1100, 100, 101, 601, 708, 742, 877, 1200, 1300) nach einem der obigen Ansprüche, wobei der Streifen (1009, 1005, 109, 125, 609, 709, 706, 889, 1312) im wesentlichen aus einem Blatt aus Schlaufenmaterial besteht, wobei sich das Harzband darüber erstreckt.

6. Beutel (1000, 1100, 100, 101, 601, 708, 742, 877, 1200, 1300) nach einem der obigen Ansprüche, wobei der Beutelkörper (1006, 1310, 119, 119"', 618, 724, 756, 876) an dem Streifen (1009, 1005, 109, 125, 609, 709, 706, 889, 1312) entlang mehrerer Kanten des Beutelkörpers gesichert ist.

7. Beutel (1000, 1100, 100, 101, 601, 708, 742, 877, 1200, 1300) nach einem der vorhergehenden Ansprüche, wobei der Streifen (1009, 1005, 109, 125, 609, 709, 706, 889, 1312) eine einstückige bzw. integrale Erstreckung von einer Seite des Beutelkörpers (1006, 1310, 119, 119"', 618, 724, 756, 876) ist.

8. Beutel (1000, 1100, 100, 101, 601, 708, 742, 877, 1200, 1300) nach einem der obigen Ansprüche, wobei der Beutelkörper (1006, 1310, 119, 119"', 618, 724, 756, 876) einen lösbaren Verschluß beinhaltet, der sich entlang der Öffnung (379, 616, 722) erstreckt.

9. Beutel (1000, 1100, 100, 101, 601, 708, 742, 877, 1200, 1300) nach Anspruch 8, wobei der Verschluß einen Rippen- bzw. Rillenverschluß umfaßt.

10. Beutel nach Anspruch 8, wobei der Verschluß einen Berührungs- bzw. Druckbefestigungsverschluß umfaßt.

11. Beutel (1100, 601, 708, 742, 1200, 1300) nach einem der vorhergehenden Ansprüche, wobei die Öffnung (379, 616, 722) zu dem Streifen (1009, 1005, 109, 125, 609, 709, 706, 889, 1312) schaut bzw. gerichtet ist.

12. Beutel (1000, 100, 101, 877) nach einem der Ansprüche 1 bis 10, wobei sich die Öffnung (379, 616, 722) entlang einer Seitenkante des Streifens (1009, 1005, 109, 125, 609, 709, 706, 889, 1312) erstreckt.

13. Beutel (1000, 1100, 100, 101, 601, 708, 742, 877, 1200, 1300) nach einem der obigen Ansprüche, wobei der Streifen (1009, 1005, 109, 125, 609, 709, 706, 889, 1312) rückstellfähig in einer Längsrichtung bzw. einem Längssinn dehnbar ist.

14. Beutel (1000, 1100, 100, 101, 601, 708, 742, 877, 1200, 1300) nach einem der obigen Ansprüche, wobei die Befestigungselemente (102) in einem diskreten bzw. gesonderten Band (1010) benachbart dem freien Ende (1003) des Streifens (1009, 1005, 109, 125, 609, 709, 706, 889, 1312) angeordnet sind.

15. Beutel (1000, 1100, 100, 101, 601, 708, 742, 877, 1200, 1300) nach Anspruch 14, wobei das gesonderte Band (1010) an die Breite des Streifens (1009, 1005, 109, 125, 609, 709, 706, 889, 1312) anschließt und sich über diese erstreckt.

16. Beutel (1000, 1100, 100, 101, 601, 708, 742, 877, 1200, 1300) nach Anspruch 14, wobei der Streifen (1009, 1005, 109, 125, 609, 709, 706, 889, 1312) einen ergreifbaren Nicht-Befestigungsbereich (D) an seinem freien Ende (1003) hinter dem Band (1010) der Befestigungselemente (102) beinhaltet.

17. Beutel (1000, 1100, 100, 101, 601, 708, 742, 877, 1200, 1300) nach einem der obigen Ansprüche, wobei das Abteil (384) vollständig innerhalb der Breite des Streifens (1009, 1005, 109, 125, 609, 709, 706, 889, 1312) definiert ist, so daß der Streifen das Abteil (384) umgibt, wenn es über den Beutelkörper (1006, 1310, 119, 119"', 618, 724, 756, 876) überlappt ist.

18. Verfahren zum lösbaren Sichern von einer oder mehreren relativ kleinen Komponente(n) an einem relativ größeren Gegenstand bzw. Objekt, wobei das Verfahren umfaßt
ein Anordnen der Komponenten in dem Abteil (384) eines Umhüllungsbeutels (1000, 1100, 100, 101, 601, 708, 742, 877, 1200, 1300) nach Anspruch 1; und
ein Umhüllen des Umhüllungsbeutels (1000, 1100, 100, 101, 601, 708, 742, 877, 1200, 1300) um den Gegenstand in einer überlappenden Weise, wobei die Befestigungselemente (102) des Umhüllungsbeutels (1000, 1100, 100, 101, 601, 708, 742, 877, 1200, 1300) an den Fasern des Umhüllungsbeutels lösbar gesichert werden, um den Umhüllungsbeutel an dem Gegenstand zu halten.

19. Verfahren nach Anspruch 18, wobei der relativ große Gegenstand ein Chassis (1220) ist und die Komponenten (1222) später mit dem Chassis (1220) zusammenzubauen sind.

20. Verfahren nach Anspruch 18, wobei der relativ große Gegenstand zu detonieren ist, und die Komponenten eine oder mehrere explosive Ladung(en) umfassen.

## Revendications

1. Sac (1000, 1100, 100, 101, 601, 708, 742, 877, 1200, 1300) comprenant un corps de sac (1006, 1310, 119, 119"', 618, 724, 756, 876), définissant là-dedans un compartiment (384) accessible à travers une ouverture (379, 616, 722) à l'une des extrémités du corps de sac (1006, 1310, 119, 119"', 618, 724, 756, 876); et
fixé au corps de sac (1006, 1310, 119, 119"', 618, 724, 756, 876) le long d'un bord de celui-ci et s'étendant depuis ceci à une extrémité distale libre (1003), une bande flexible (1009, 1005, 109, 125, 609, 709, 706, 889, 1312) d'une largueur suffisante pour recouvrir au moins une majorité du compartiment (384), la bande portant un groupement d'éléments d'attache (102) sur un côté opposé de celui-ci, chaque élément d'attache (102) ayant une tige s'étendant intégralement depuis une bande de résine qui s'étend à travers la bande (1009, 1005, 109, 125, 609, 709, 706, 889, 1312);
la bande (1009, 1005, 109, 125, 609, 709, 706, 889, 1312) étant d'une longueur suffisante pour envelopper un objet, la bande (1009, 1005, 109, 125, 609, 709, 706, 889, 1312) recouvrant le corps de sac (1006, 1310, 119, 119"', 618, 724, 756, 876) afin d'engager des fibres exposées du sac (1000, 1100, 100, 101, 601, 708, 742, 877, 1200, 1300) avec les éléments d'attache (102), pour fixer le sac à l'objet.

2. Sac (1000, 1100, 100, 101, 601, 708, 742, 877, 1200, 1300) selon la revendication 1, dans lequel la bande de résine encapsule des particularités de surface de la bande (1009, 1005, 109, 125, 609, 709, 706, 889, 1312) pour former un stratifié inséparable.

3. Sac (1000, 1100, 100, 101, 601, 708, 742, 877, 1200, 1300) selon la revendication 1, dans lequel la bande de résine est une région intégrale de la bande (1009, 1005, 109, 125, 609, 709, 706, 889, 1312), la bande étant une feuille unitaire de la résine et s'étendant au-delà de la série d'éléments d'attache.

4. Sac (1000, 1100, 100, 101, 601, 708, 742, 877, 1200, 1300) selon l'une quelconque des revendications précédentes, dans lequel la bande (1009, 1005, 109, 125, 609, 709, 706, 889, 1312) s'étend au-delà du bord du corps de sac (1006, 1310, 119, 119"', 618, 724, 756, 876) à une distance étant environ plus de deux fois la largueur du corps de sac (1006, 1310, 119, 119"', 618, 724, 756, 876), mesurée depuis le même bord dans un sens opposé.

5. Sac (1000, 1100, 100, 101, 601, 708, 742, 877, 1200, 1300) selon l'une quelconque des revendications précédentes, dans lequel la bande (1009, 1005, 109, 125, 609, 709, 706, 889, 1312) consiste essentiellement en une feuille de matériel de boucle, la bande de résine s'étendant à travers de ceci.

6. Sac (1000, 1100, 100, 101, 601, 708, 742, 877, 1200, 1300) selon l'une quelconque des revendications précédentes, dans lequel le corps de sac (1006, 1310, 119, 119"', 618, 724, 756, 876) est fixé à la bande (1009, 1005, 109, 125, 609, 709, 706, 889, 1312) le long de multiples bords du corps de sac.

7. Sac (1000, 1100, 100, 101, 601, 708, 742, 877, 1200, 1300) selon l'une quelconque des revendications précédentes, dans lequel la bande (1009, 1005, 109, 125, 609, 709, 706, 889, 1312) représente une extension intégrale de l'un côté du corps de sac (1006, 1310, 119, 119"', 618, 724, 756, 876).

8. Sac (1000, 1100, 100, 101, 601, 708, 742, 877, 1200, 1300) selon l'une quelconque des revendications précédentes, dans lequel le corps de sac (1006, 1310, 119, 119"', 618, 724, 756, 876) inclut une fermeture détachable s'étendant le long de l'ouverture (379, 616, 722).

9. Sac (1000, 1100, 100, 101, 601, 708, 742, 877, 1200, 1300) selon la revendication 8, dans lequel la fermeture comprend une fermeture de nervure et de cannelure.

10. Sac selon la revendication 8, dans lequel la fermeture comprend une fermeture réglable d'attache de contact.

11. Sac (1100, 601, 708, 742, 1200, 1300) selon l'une quelconque des revendications précédentes, dans lequel l'ouverture (379, 616, 722) se trouve face à la bande (1009, 1005, 109, 125, 609, 709, 706, 889, 1312).

12. Sac (1000, 100, 101, 877) selon l'une quelconque des revendications 1 à 10, dans lequel l'ouverture (379, 616, 722) s'étend le long du bord de l'un côté de la bande (1009, 1005, 109, 125, 609, 709, 706, 889, 1312).

13. Sac (1000, 1100, 100, 101, 601, 708, 742, 877, 1200, 1300) selon l'une quelconque des revendications précédentes, dans lequel la bande (1009, 1005, 109, 125, 609, 709, 706, 889, 1312) est étirable d'une façon résiliente dans un sens longitudinal.

14. Sac (1000, 1100, 100, 101, 601, 708, 742, 877, 1200, 1300) selon l'une quelconque des revendications précédentes, dans lequel les éléments d'attache (102) sont disposés dans une bande distincte (1010) adjacent au bout libre (1003) de la bande (1009, 1005, 109, 125, 609, 709, 706, 889, 1312).

15. Sac (1000, 1100, 100, 101, 601, 708, 742, 877, 1200, 1300) selon la revendication 14, dans lequel la bande distincte (1010) est ininterrompue et s'étend à travers la largueur de la bande (1009, 1005, 109, 125, 609, 709, 706, 889, 1312).

16. Sac (1000, 1100, 100, 101, 601, 708, 742, 877, 1200, 1300) selon la revendication 14, dans lequel la bande (1009, 1005, 109, 125, 609, 709, 706, 889, 1312) inclut une région graspable et non-attachable (D) à son bout libre (1003), au-delà de la bande (1010) des éléments d'attache (102).

17. Sac (1000, 1100, 100, 101, 601, 708, 742, 877, 1200, 1300) selon l'une quelconque des revendications précédentes, dans lequel le compartiment (384) est défini entièrement dans la largueur de la bande (1009, 1005, 109, 125, 609, 709, 706, 889, 1312), de façon à ce que la bande enveloppe le compartiment (384) quand elle est recouverte à travers le corps de sac (1006, 1310, 119, 119"', 618, 724, 756, 876).

18. Procédé de fixer détachablement un ou plusieurs composants relativement petits à un objet relativement plus grand, procédé comprenant
placer des composants dans le compartiment (384) d'un sac-enveloppe (1000, 1100, 100, 101, 601, 708, 742, 877, 1200, 1300) selon la revendication 1;
et envelopper le sac-enveloppe (1000, 1100, 100, 101, 601, 708, 742, 877, 1200, 1300) de façon recouvrante autour de l'objet, fixant détachablement les éléments d'attache (102) du sac-enveloppe (1000, 1100, 100, 101, 601, 708, 742, 877, 1200, 1300) aux fibres du sac-enveloppe, afin de maintenir le sac-enveloppe à l'objet.

19. Procédé selon la revendication 18, dans lequel l'objet relativement grand représente un châssis (1220), et les composants (1222) doivent être rassemblés ultérieurement au châssis.

20. Procédé selon la revendication 18, dans lequel l'objet relativement grand doit être détoné, et dans lequel les composants comprennent un ou plusieurs charges explosives.
